# EUROPÄISCHE PATENTSCHRIFT

(11) **EP 1 392 174 B1**
(45) Veröffentlichungstag und Bekanntmachung des Hinweises auf die Patenterteilung: **14.07.2010**
(21) Anmeldenummer: 02722268.6
(22) Anmeldetag: 25.03.2002
(51) Int. Cl.: A61B 17/00, B25J 9/16, G05B 19/401

(54) **VERFAHREN UND GERÄTESYSTEM ZUM MATERIALABTRAG ODER ZUR MATERIALBEARBEITUNG**
METHOD AND DEVICE SYSTEM FOR REMOVING MATERIAL OR FOR WORKING MATERIAL
PROCEDE ET APPAREIL POUR EXTRAIRE DE LA MATIERE OU POUR TRAVAILLER DE LA MATIERE

(30) Priorität: 26.03.2001 DE 10114910; 28.03.2001 DE 10115170; 06.04.2001 DE 10117403
(43) Veröffentlichungstag der Anmeldung: 03.03.2004
(73) Patentinhaber: ALL-OF-INNOVATION Gesellschaft mit beschränkter Haftung, 85737 Ismaning (DE)
(72) Erfinder: LÜTH, Tim, 85737 Ismaning (DE); BIER, Jürgen, 10719 Berlin (DE); BIER, Angelika, 10719 Berlin (DE); HEIN, Andreas, 26133 Oldenburg (DE); SCHERMEIER, Olaf, 14059 Berlin (DE)
(74) Vertreter: Hengelhaupt, Jürgen
(86) Internationale Anmeldenummer: PCT/EP2002/003334
(87) Internationale Veröffentlichungsnummer: WO 2002/076302

(56) Entgegenhaltungen:
- WO-A-00/64367
- WO-A-96/11624
- WO-A-96/11624
- WO-A-97/12559
- US-A- 5 297 238
- US-A- 5 408 409
- US-A- 5 408 409
- US-A- 5 630 431
- US-A- 5 630 431
- US-A- 5 825 017
- US-A- 6 021 343
- US-A- 6 112 113

## Beschreibung

Die Erfindung betrifft ein Verfahren und Gerätesystem zum Material - oder Gewebeabtrag oder zur Material- oder Gewebebearbeitung und ist anwendbar in der Medizin und Zahnmedizin sowie für verschiedenste Arten der Materialbearbeitung in unterschiedlichen Anwendungsgebieten und Modellarbeiten gemäß dem Oberbegriff des Anspruchs 1.

Das erfindungsgemäße Verfahren und das Gerätesystem ist beispielsweise für das kontrollierte Anordnen und Führen von Handstücken sowie die Zu- und Abschaltung, Regelung oder Parameterisierung der Effektorenergie in der Chirurgie und Zahnheilkunde zum optimalen Gewebeabtrag als Vorbereitung für die Konservierung und das Inserieren von Implantaten, Inlays und Onlays anwendbar. Es lassen sich auch Schnitte mit hoher Präzision setzen.

Zum gegenwärtigen Zeitpunkt sind für den Gewebeabtrag in der Medizin überwiegend Instrumente wie Bohrer, Fräsen und Sägen im Einsatz, die in das Spannfutter eines medizinischen Handstücks eingesetzt werden. Vereinzelt kommen auch Lasersysteme zum Einsatz, die Weich- und Hartgewebe trennen und/oder abtragen können.

Beim Gewebeabtrag werden Gewebeschnitte, Gewebeöffnungen und Gewebelöcher (Kavitäten) oder Durchgänge erzeugt, die medizinischen Kriterien (z.B. Restgewebe ist tumorfrei, bakterienfrei, nicht kariös, oder Restgewebe hat hohe Festigkeit) und/oder weiteren Kriterien (z.B. die Geometrie des entfernten Gewebes besitzt eine besondere Passform zum Einfügen eines Gegenstücks) genügen sollen.

Es sind aus der Messtechnik Koordinatenmeßsysteme bekannt, mit denen die Lage (Position und Orientierung) eines Werkzeugs relativ zu einem Referenzkoordinatensystem vermessen werden kann.

Es sind aus der computerassistierten Chirurgie medizinische Navigationssysteme bekannt, mit denen die Lage (Position und Orientierung) eines Instrumentes relativ zu Patientengewebe angezeigt werden kann, nachdem das Gewebe registriert worden ist.

Auch sind aus der roboterassistierten Chirurgie medizinische Robotersysteme bekannt, mit denen die Instrumente von einem Roboter auf vorgeplanten Bahnen bewegt werden können, damit beispielsweise eine Bohrung an eine bestimmte Lage (Position und Orientierung) gelangt oder eine Kavität ausgebohrt wird, die eine besondere geometrische Form hat.

Weiterhin sind aus der roboterassistierten Chirurgie medizinische Interaktionssysteme bekannt, bei denen die Instrumente an einer passiven (aktiv bremsend) oder aktiven (aktiv bewegend) Kinematik befestigt sind, jedoch vom Arzt manuell durch direktes Führen des Instruments oder der Kinematik innerhalb von definierten Volumen, auf definierten Flächen und entlang definierter Bahnen (Geraden, Kurven) bewegt werden können, um beispielsweise eine Bohrung an einer bestimmten Lage (Position und Orientierung) zu setzen oder eine Kavität auszubohren, die eine besondere geometrische Form haben soll.

Ebenso sind aus der roboterassistierten Chirurgie medizinische Telemanipulationssysteme bekannt, bei denen die Instrumente an einer aktiven Kinematik (Slave-Manipulator) befestigt sind, jedoch vom Arzt weitgehend über eine angekoppelte Eingabekinematik (Master-Manipulator) manuell innerhalb von definierten Volumen, auf definierten Flächen und entlang definierter Bahnen (Geraden, Kurven) bewegt werden können, um beispielsweise einer Bohrung an einer bestimmten Lage (Position und Orientierung) zu setzen oder eine Kavität auszubohren, die eine besondere geometrische Form haben soll.

In der Zahnheilkunde sind Handscanner bekannt, die über Streifenprojektion oder andere Verfahren ein 3D Oberflächenmodell mit hoher Genauigkeit vermessen können. Einem Arzt ist es bisher mit einem manuell geführten Instrument nicht möglich, Gewebeabtragungen so durchzuführen, dass die Lage und/oder die Geometrie der Gewebeabtragungen vorab oder dynamisch definierten medizinischen Kriterien (z.B. das Restgewebe ist tumorfrei, bakterienfrei, nicht kariös, oder Restgewebe hat hohe Festigkeit) oder geometrischen Kriterien (z.B. das Restgewebe oder entnommene Gewebe besitzt eine besondere Passform zum Einfügen eines Gegenstücks) mit einer hohen Güte entspricht.

Dies hängt mit der mangelnden Fähigkeit des Menschen zusammen, seine Hände in einem Referenzkoordinatensystem räumlich präzise auszurichten.

Auch mit einem Navigationssystem ist es einem Arzt mit einem manuell geführten Instrument bisher nicht möglich, Gewebeabtragungen so durchzuführen, dass die Lage und/oder die Geometrie der Gewebeabtragungen vorab oder dynamisch definierten medizinischen Kriterien (z.B. das Restgewebe ist tumorfrei, bakterienfrei, nicht kariös, oder Restgewebe hat hohe Festigkeit) oder geometrischen Kriterien (z.B. das Restgewebe oder entnommene Gewebe besitzt eine besondere Passform zum Einfügen eines Gegenstücks) mit einer hohen Präzision entspricht.

Nichttaktile gewebeabtragende Effektoren wie beispielsweise Laserstrahlen erlauben es dem Benutzer bei einer manuellen Bearbeitung eines Hartgewebes nicht gefühlsmäßig die Form des abgetragenen Gewebes oder der entstehenden Passform zu erfassen. Es können daher manuell keine Passformen hergestellt werden, die bestimmten Kriterien (z.B. zylindrisch) genügen.

Robotergesteuerte, telemanipulierte oder interaktiv robotergeführte Instrumenteneinsätze sind immer mit einem erheblichen Geräteaufwand verbunden, was zu einer Kostensteigerung führt.

Darüber hinaus erfordert es auch ein hohes Ausbildungs- und Motivationsniveau des beteiligten medizinischen Personals sowie des Pflegepersonals. Es muss erheblicher Einarbeitungs- und Installationsaufwand geleistet werden. Die Operationen dauern oftmals länger als ohne Roboter.

Die Patienten müssen fixiert werden, um mit einem Roboter die gewünschte Qualität zu erreichen.

In der Zahnheilkunde werden oftmals versehentlich benachbarte Strukturen beim Umgang mit einem gewebeabtragenden Sensor und/oder Instrument unerwünscht verletzt. Es ist selbst navigationsunterstützt nicht möglich, eine Kavität sauber auszuformen. Es können keine vorgefertigten Implantate sauber eingepasst werden. Es ist nicht möglich, später passende Inlays, Onlays oder Brücken vorab anzufertigen. Es ist nicht möglich, eine später passende Suprakonstruktion vorab anzufertigen und diese perfekt einzupassen. Es ist nicht möglich, Standardinlays, Onlays, oder Brücken zu verwenden, die in sehr hoher Qualität bei einem Implantathersteller oder vergleichbaren Herstellern angefertigt werden. Es ist nicht möglich, Kavitäten sauber so auszuformen, dass sie bestimmten medizinischen Kriterien (z.B. Abstand zu bakteriösen, tumorösen Gewebe) entsprechen. Es ist nicht möglich, Kavitäten sauber so auszuformen, dass sie bestimmten herstellungstechnischen Kriterien (z.B. Formgebung des Passkörpers für die Herstellung mit 3-Achs-Fräsen) entsprechen. Es ist nicht möglich, Kavitäten sauber so auszuformen, dass sie bestimmten Kriterien zur Integration von Passkörpern (Einschub, einstecken, verdrehbar sichern), entsprechen. Es ist nicht möglich, die Kavitäten so auszuformen, dass sie Kombinationen der Kriterien entsprechen. Es ist nicht möglich, einen manuell ausgeführten Gewebeabtrag (z.B. am Modell) zu erfassen, zu speichern und als "Vorlage" für einen Gewebeabtrag mit identischer Form an dem selben oder an einem anderen Objekt (z.B. Patientengewebe) zu verwenden).

In der Weichgewebschirurgie können keine Schnitte so gelegt werden, so dass sie bestimmten medizinischen Kriterien (z.B. Abstand zu bakteriösen, tumorösen Gewebe) und/oder Kriterien für die Integration von Transplantaten und Implantaten (z.B. Brustimplantate nach Gewebsentfernung) entsprechen.

In der Knieendoprothethik können keine Mehrfachschnitte ohne Fixierung oder kinematischer Führung mit sauber zueinander definierten Schnittflächen hergestellt werden.

In der Wirbelsäulenchirurgie können keine Dekompressionen und Pedikelschraubeninserierung ohne Fixierung des Gewebes und/oder kinematischer Führung der Instrumente durchgeführt werden.

Ein weiterer Nachteil der bekannten Lösungen besteht darin, dass die Navigationssysteme gemäß dem Stand der Technik keine Werkzeuge verwenden können, deren Übergangsmatrix nicht vorab bekannt ist. Dies schränkt den Nutzer auf ein Werkzeugset einer bestimmten Firma ein. Der Nutzer ist nicht in der Lage, ein neues Werkzeug unkompliziert einzumessen. Das Einniessen ist mit mindestens einem Knopfdruck verbunden. Werden die Werkzeuge eines Instruments wie beispielsweise ein Handstück gewechselt, besteht die große Gefahr der Benutzung eines nicht registrierten Werkzeugs. Dies kann zu Verletzungen des Patienten führen, da die Positions- und Winkelangaben falsch sind, was jedoch nicht erkannt werden kann.

Handstücke insbesondere für die computerassistierte Zahnheilkunde werden in verschiedenen Veröffentlichungen beschrieben. In der Zahnheilkunde gibt es gegenwärtig zwei Verfahren, die eine Markierung des Handstücks für eine räumliche Referenzierung erfordern. Dies ist zum einen manuelles Bohren mit navigierter Lageorientierung, zum anderen Bohren mit einem kinematischen Aufbau, z.B. Roboter.

Die US 4824367 beschreibt eine Vorrichtung zur Anzeige der parallelen Ausrichtung eines dentalen Handstückes bestehend aus einem Winkelanzeiger zur Generierung von elektrischen Winkelsignalen, welche die Ausrichtung eines Fräskopfes anzeigen, der mit einem dentalen Handstück betrieben wird, Einstellungselemente zum Einstellen von elektrischen Referenzsignalen, welche die Position einer voreingestellten Achse anzeigen, Warnelementen, welche Warnsignale aussenden, wenn das Winkelsignal außerhalb eines voreingestellten Bereichs liegt.

Die US 5017139 beschreibt eine Vorrichtung mit einem dentalen/ medizinischen Operationsinstrument zur Gewinnung von dreidimensionalen Konturinformationen, bestehend aus einer Mehrzahl von Armsegmenten, welche sequentiell miteinander verbunden sind, so dass eine Struktur mit einem vorderen und einem hinteren Ende entsteht, einem ersten Befestigungselement zur Befestigung des ersten Endes der Struktur an einer festen Plattform und einem zweiten Befestigungselement zum Anbringen eines Operationsinstrumentes am zweiten Ende der Struktur, einer Mehrzahl von Encodern, wobei jeder der Encoder mit einem der Armsegmente verbunden ist, um ein elektrisches Signal, welches die Position der einzelnen Segmente anzeigt, zu generieren, wodurch die Position des Operationsinstrumentes kontinuierlich verfolgt werden kann.

Die US 6000939 beschreibt eine Vorrichtung zum präzisen Ausrichten von Zahnbohrern bestehend aus Orientierungselementen zum Anbau an ein dentales Handstück, welche ein Signal des Bohrwinkels generieren und Orientierungselementen zum Anbau an eine Zahnstruktur, welche ein Signal des Zahnwinkels generieren und Vergleichselemente, welche Warnsignale aussenden, wenn die Differenz der Winkelsignale außerhalb eines voreingestellten Bereichs liegt.

Die EP 0 741 994 A1 beschreibt ein Verfahren zur Darstellung des Kiefers einer Person, welches aus folgenden Schritten besteht: Einführung einer Vorrichtung mit Markierungspunkten zur Lagebestimmung in die Mundhöhle der Person, Erstellen mindestens einer Aufnahme des Kiefers mit einem bildgebenden Verfahren, wobei die Markierungspunkte mit aufgenommen werden, Identifikation der Markerpunkte, wobei zur Darstellung folgende Punkte durchgeführt werden: Anbringen eines 3D-Sensors auf der Außenseite des betreffenden Kiefers, Neuerliches Einführen der Vorrichtung zur Lagebestimmung, falls diese inzwischen entnommen ist, in die Mundhöhle in gleicher Lage wie bei der Erstellung der Aufnahme, wobei die Vorrichtung mit einem 3D-Sensor ausgestatten ist, Bestimmung der lagemäßigen Beziehung zwischen dem 3D-Sensor der Vorrichtung und dem 3D-Sensor auf der Außenseite des Kiefers, Entfernen der Vorrichtung zur Lagebestimmung, Erzeugen einer Überlagerung eines optischen Bildes des Kiefers mit dem Datensatz in lagerichtiger Zuordnung Truppe beschreibt das Verfahren auch für die Darstellung eines Modells des Kiefers und für die Darstellung des Modells des Kiefers und dem Kiefer. Truppe beschreibt weiterhin ein Verfahren zur Darstellung des Kiefers und/oder eines Modells davon, wobei zusätzlich eine fotografische oder Video-Aufnahme des Kiefers bzw. des Modells angefertigt wird, die mit der Aufnahme des Bildgebenden Verfahrens überlagert wird.

Ultraschall-, optische oder mechanische Sensoren eingesetzt werden.

Aus US 5688118 ist ein System zur Schulung von Zahnärzten in der Herstellung von Kavitäten in Zähnen bekannt. Dabei wird ein in einem Behandlungsstuhl ein menschlicher Phantomtorso mit einem Modellkiefer positioniert. Der Schüler arbeitet mit einer speziellen Schulungseinheit aus pneumatischem Bohrerantrieb und Handstück, die in ihrem Aufbau und in ihrer Betriebsweise bzw. Anwendungsweise von einem "echten" Behandlungseinheit für den Patientenbetrieb abweicht. Über ein 3D-Messsystem können die Position und Orientierung von "Handstück" bzw. "Bohrer" sowie eines "Spiegels" im Raum gemessen werden. Das System hat die Aufgabe, dreidimensionale Bilder eines Modellkiefers mit Zähnen am Bildschirm darzustellen und die Positionen des vom Schüler gehaltenen zahnärztlichen Handstücks am Bildschirm relativ zu dem Bilddaten des Phantoms darzustellen. Es hat die Aufgabe das "Bild" eines zahnärztlichen Spiegels aus den Modelldaten zu berechnen und darzustellen. Es hat die Aufgabe die Ausbildungszeit eines Zahnarztes in der Ausbildung bei der Präparation von Kavitäten zu verkürzen. Es soll das Geräusch und das Gefühl wie beim echten Bohren einer Zahnkavität vermitteln. Das Gerät wirkt auf den Schüler, damit dieser später in einer Behandlungssituation mit einem richtigen Patienten und einer richtigen Behandlungseinheit ohne Navigationshilfen akustische, taktile und visuelle Informationen richtig interpretiert und seine Handlung danach richtet. Der Schüler hat die Aufgabe in einen künstlichen Zahn des Phantoms eine Kavität zu bohren unter Berücksichtigung einer von dem Schulungskonzept vorgegeben Zahnsituation. Mit einem Ventil kann die Druckzufuhr des pneumatischen Antriebs eingestellt werden, um dem Schüler bestimmte Eigenschaften einer Behandlungssituation akustisch und visuell zu vermitteln. Die Leistung des Bohrers wird reduziert, wenn ein hartes Zahnmaterial simuliert werden soll und wird gesteigert, wenn ein weiches Zahnmaterial simuliert werden soll. Die Steuerung richtet sich generell nach programmierten geometrischen modellierten Eigenschaften des simulierten Zahnmodells. Das Gesamtsystem kann aus ergonomischen Gesichtspunkten eine optische Ähnlichkeit mit einem zahnärztlichen Behandlungssystem besitzen. Das System ist von seinem Konzept und seiner Wirkungsweise nicht als Behandlungssystem einsetzbar.

Aus US 5257203 ist ein Verfahren zur Ansteuerung einer Werkzeugmaschine beschrieben, mit der sich unter anderem zahnärzliche Modellarbeiten durchführen lassen. Eine derartige Maschine stellt eine hervoragende Ergänzung zu der eigenen Erfindung dar. Diese Maschine wird jedoch nicht am Patienten eingesetzt und ist nicht in der Lage Hinterschneidungen der Kavitäten am Patienten nachträglich zu kompensieren.

Aus US 5725376 ist das Verfahren zur Herstellung von Bohrschablonen beschrieben. Diese Verfahren haben in der Praxis den entscheiden Nachteil, dass die Fixierung der Bohrschablone auf der Mundschleimhaut schwierig ist und gerade an der Stelle an der gebohrt werden soll eine Schablone zur Führung des Handstücks liegt. Das Verfahren kann nicht zur beliebigen Ausformung von Kavitäten angewendet werden.

Aus DE 19534590 A1 ist ein Verfahren zur Ablation von Zahnhartsubstanzen beschrieben. Dabei wird die Laserleistung in Abhängigkeit vom Abstand zwischen Laserhandstück und Gewebe geschaltet. Es lassen sich keine Gewebeabtragungen mit besonderen geometrischen Eigenschaften erzeugen.

Die DE 199 02 273 A1 beschreibt eine Vorrichtung zur intraoperativen Bestimmung einer Platzierung von Dentalimplantaten im Kieferknochen mittels eines Navigationssystems, durch das die momentane Implantatbohrerposition in einer dreidimensionalen Röntgenaufnahme abgebildet wird und die Position im Raum mittels eines befestigten dynamischen Referenzrahmens bestimmt wird, **dadurch gekennzeichnet, dass** der dynamische Referenzrahmen mindestens aus einem Befestigungselement an den Zähnen und/oder Kiefer und einem zugeordneten lösbaren Element mit dem dynamischen Referenzrahmen besteht. Das Verfahren wurde jedoch bereits 1998 an der Charité verwendet und publiziert.

Die US 5332391 beschreibt eine Vorrichtung zur Unterstützung einer Vielzahl von dentalen Handstücken wobei jedes einen anderen Winkel der Bohrachse besitzt zur normalen Ausrichtung der Bohrachse zu der Okklusionsebene der Zähne, bestehend aus: Einem Halter zum Führen eines dentalen Handstückes, eine Verbindung in Form einer Parallelstruktur mit einem freien Ende an dem ein Drehpunkt befestigt ist um die Orientierung des Bohrers zur Okklusionsebene konstant zu halten und Elemente die neben dem Drehpunkt angeordnet sind zur Verbindung des Halters mit dem Drehpunkt, wobei der Halter abnehmbar ist.

Die US5989024 beschreibt einen Apparat zur Zusammenwirkung mit einem angetriebenen Werkzeug mit einer longitudinalen Achse, welcher die Achse des Werkzeuges konstant hält, wenn das Werkzeug im Raum bewegt wird, bestehend aus: einem einstellbaren Arm mit zwei Enden, einer Klammeranordnung zur Befestigung des Werkzeuges an einem der Enden, einer Basis an dem anderen Ende, welche an einem Arbeitsstück befestigt werden kann, wobei der Arm einen ersten Teil enthält, der Bewegungen entlang der longitudinalen Achse des Werkzeuges erlaubt.

Die US 5281136 beschreibt einen Apparat zur Unterstützung eines dentalen Bohrers bestehend aus: Einem beweglichen Arm welcher an einem festen Bezugspunkt fixiert werden kann und wobei der Arm sich am Ende eines dentalen Bohrers befestigen lässt, wobei der Arm so aufgebaut ist, dass er die Achse des Bohrers konstant normal zu einer voreingestellten Arbeitsebene hält, Komponenten zur Stabilisierung des Kopfes und des Kiefers des Patienten, bestehend aus einer Kopflagerung, die an einem Stuhl befestigt werden kann und Elementen zur Fixierung des Kiefers an der Kopflagerung.

Die US 5575646 beschreibt eine Vorrichtung zur Unterstützung eines dentalen Bohrers bestehend aus: einem Lager, einem Arm mit zwei viereckigen Elementen, welche in Serie miteinander verbunden sind während eines der Vierecke mit dem Lager und ein anderes mit einem Teil verbunden ist, welches ein dentales Bohrinstrument hält, so dass die Achse des Bohrinstrumentes konstant bleibt, einem Einstellelement zur Einstellung der Richtung der Arbeitsachse, wobei das Lager mit Fixierungselementen ausgestattet ist, um es mit der Rückenlehne eines Stuhles zu verbinden, wobei die viereckigen Elemente mit 90 Grad zueinander ausgerichtet sind, so dass ein Element sich über dem Patienten befindet und ein anderes sich vor dem Patienten befindet.

Die US 6030211 beschreibt einen Führungsapparat bestehend aus: Einem Schlitten, der an einem Punkt befestigt ist, einem Zwischenteil, welches an einem Ende an dem Schlitten befestigt ist und in einer ersten longitudinalen Koordinate z bewegt werden kann und ein anderes Ende zur Aufnahme eines Verbindungsarmes über ein Drehgelenk besitzt, einem Arbeitskopf an diesem Verbindungsarm, welcher einen Instrumentenhalter hält und zwei Elemente zur Bewegung des Instrumentenhalters in zwei weitere longitudinale Achsen x und y, wodurch der Instrumentenhalter in x, y, z und einer rotatorischen Achse beweglich ist.

Die WO98/40030 beschreibt ein System zur Übertragung der simulierten Position von Dentalimplantaten von einem Röntgengerät auf einem Roboter, welcher genutzt wird, um in einen Abdruck des Kiefers eines Patienten zu bohren. Das System beinhaltet ein mechanisches Lager sowie Elemente zur Befestigung des Abdruckes am Lager in einer reproduzierbaren Lage, der Abdruck beinhaltet mindestens zwei rechtwinklige Elemente, die in einer Röntgenaufnahme sichtbar sind.

Dokumente W09611624 A2 und US5408409 offenbaren ein Verfahren zum Materialabtrag oder zur Materialbearbeitung mittels mindestens eines manuell geführten Effektors (medizinisches Handstück, mit z.B. Bohrer, Laser, Fräse) oder Effektoren von Robotern, unter Einsatz eines Navigationssystems. In beiden Dokumenten werden Sicherheitsfunktionen offenbart, wobei der Effektor in Abhängigkeit von einem vorgegebenen Arbeitsvolumen ("safe zone") in Ein/Aus-Funktion geschaltet werden.

Beim gegenwärtigen Stand der Technik gibt es keine Möglichkeit die Handstücke des Zahnarztes nachträglich bei- nur kleinen Modifikationen mit einer Markierung zu versehen, so dass sie problemlos mit einem Navigationssystem verwendet werden können. Es gibt spezielle Handstücke für diese Anwendung, die jedoch teuer vom Zahnarzt erworben werden müssen. Sie erlauben keine Verwendung normaler Turbinen und keine einfache Trennung von Turbine und Handstück.

Der Zahnarzt muss sowohl "normale" als auch "navigierbare" Handstücke vorhalten.

Aufgabe der Erfindung ist es, die bekannten Nachteile des Standes der Technik zu vermeiden und ein Verfahren und ein Gerätesystem zu schaffen, welche es dem Benutzer ermöglichen, durch gezielte Beeinflussung des Effektors kontrolliert Material oder Gewebe abzutragen oder zu bearbeiten und beim Abtrag dabei nicht zu viel und nicht zu wenig Material oder Gewebe zu entfernen. Das Gerätesystem soll mit einfachen Mitteln den Effektor steuern oder regeln bzw. ein- oder ausschalten.

Diese Aufgabe wird erfingdungsgemäß durch ein Verfahren und Gerätesystem gemäß den Merkmalen der Ansprüche 1 und 17 gelöst.

Zweckmäßige Ausgestaltungen der Erfindung sind in den jeweiligen Unteransprüchen enthalten.

Ein besonderer Vorteil der Erfindung besteht darin, dass innerhalb kürzester Zeit ein exakter Materialabtrag oder eine hochgenaue reproduzierbare Materialbearbeitung realisiert werden kann, indem Daten zur Position und/oder Orientierung des Effektors und deren Änderungen relativ zur Lage mindestens eines Referenzkörpers erfasst, gespeichert und rechentechnisch verarbeitet werden und Befehle zur Steuerung und/oder Regelung auslösen derart, dass in Abhängigkeit eines vorgegebenen Arbeitsvolumens und/oder Materialabtragsvolumens und/oder Materialrestvolumens der Effektor in Ein/Aus-Funktion geschaltet oder in der Ein-Funktion in seiner Leistung und/oder Parameterisierung gesteuert und/oder geregelt wird.

Multivalente Einsatzgebiete erschließen sich dadurch, dass die Lage und Geometrie der erreichten Objektoberfläche erfasst und für weitere Bearbeitungsvorgänge am selben oder anderen Objekten gespeichert wird.

Das Verfahren und das Gerätesystem zum Materialabtrag oder zur Materialbearbeitung beruht auf der Basis, dass Material von einem Objekt zur Erfüllung von mindestens einem Kriterium bearbeitet oder abgetragen wird und vorzugsweise die Leistung und/oder Form und/oder Lage des angeordneten und/oder geführten materialbearbeitenden oder materialabtragenden Effektors so gesteuert oder geregelt wird, dass Kriterien möglichst optimal erfüllt werden können und die Lage und Geometrie der erreichten Objektoberfläche erfasst und für weitere Bearbeitungsvorgänge am selben oder anderen Objekten gespeichert wird und vorzugsweise die Herstellung, Bearbeitung von einzusetzende Passkörpern sowie der Zeitraum zwischen Materialabtrag und Einsatz von hergestellten oder vorhandenen Passkörpern verkürzt wird. Das Verfahren und das System sind beispielsweise für das kontrollierte Anordnen, Führen von Handstücken sowie die Zu- und Abschaltung der Effektorenergie in der Chirurgie und Zahnheilkunde zum optimalen Gewebeabtrag als Vorbereitung für die Konservierung und das Inserieren von Implantaten, Inlays und Onlays vorteilhaft anwendbar. Es lassen sich auch Schnitte mit hoher Präzision setzen.

Es ist möglich, hinsichtlich geometrischer Ansprüche saubere präzise Schnitte zu legen, Bohrungen zu setzen, Kavitäten- oder Stümpfe zu fräsen und die Bewegungen sauber zu vermessen.

In der Zahnmedizin ist es selbst frei Hand möglich, Löcher für Implantate so exakt zu bohren, als ob eine Führungskinematik verwendet worden wäre. In der Zahnmedizin ist es dann möglich, ein Inlay, Onlay oder eine Brücke vorab anzufertigen und die Kavitäten so auszuformen, dass die vorhandenen Inlays oder Onlays perfekt in die Kavitäten oder auf den Stumpf passen. Dadurch entfällt die vor Ort Herstellung eines Inlays oder Onlays. Inlays und Onlays können deutlich preiswerter und mit höherer Qualität zentral hergestellt und gelagert werden. Der Zeitraum zwischen Ausformen und Versorgung sinkt erheblich.

In der Zahnmedizin ist es möglich, gleichzeitig das Gewebe unter medizinischen Gesichtspunkten zu entfernen und parallel dazu eine Kavität oder einen Stumpf zu formen, der das Fügen mit einem Passkörper (ohne Hinterschneidungen), bei maximalem Gewebeerhalt erlaubt. Es können komplizierte Geometrien erreicht werden.

In der Weichgewebechirurgie lassen sich bei dem Trennen von komplizierten Gewebestrukturen (Viszeral-chirurgie) manuell perfekte Gewebeabtrennung erreichen, wenn gleichzeitig ein Gewebepositionsmeßsystem z.B. auf der Basis von elektromagnetischen Reflektoren verwendet wird. Kostenintensive mechanische Aufbauten für die Führung der Instrumente können entfallen. Es können auch ohne den Einsatz von Robotern vergleichbare Ergebnisse bei einer manuellen Instrumentenführung erreicht werden. Dies ist extreme Verbesserung der gegenwärtigen Situation. Medizinische Fräsroboter können in Ihrer Funktion weitgehend ersetzt werden.

Es kann auch bei dem Gewebeabtrag die Geometrie des Gewebeabtrags vermessen werden und diese Geometrie dann mehrfach verwendet werden. Dies hat Vorteile bei der Übertragung von Modellarbeiten auf andere Gewebearten. Es können auch Formen an einer Achse oder als Volumenmodelle gespiegelt werden (Positiv, Negativform).

Die Vorlage kann auch an einer oder mehreren Achsen gespiegelt werden. Teile der Geometrie können von Negativin Positivformen gespiegelt werden.

Ein Nutzer kann mit einem manuell geführten Instrument Gewebeabtragungen so durchzuführen, dass die Lage und/oder die Geometrie der Gewebeabtragungen vorab oder dynamisch definierten medizinischen Kriterien (z.B. das Restgewebe ist tumorfrei, bakterienfrei, nicht kariös, oder Restgewebe hat hohe Festigkeit) oder geometrischen Kriterien (z.B. das Restgewebe oder entnommene Gewebe besitzt eine besondere Passform zum Einfügen eines Gegenstücks) mit einer hohen Güte entspricht. Die mangelnde Fähigkeit des Menschen seine Hände in einem Referenzkoordinatensystem räumlich präzise auszurichten wird ausgeglichen.

In der Zahnhelilkunde können vorab angefertigte Suprakonstruktionen, Inlays, Onlays oder Brücken verwendet werden. Es können komplizierte Geometrieformen manuell hergestellt werden. Es können CAD-Daten des Abtrags erstellt werden. Die Geometriedaten können zur Herstellung von Implantaten oder zum Heraustrennen von Transplantaten verwendet werden. Die Geometriedaten können zum Qualitätsnachweis verwendet werden.

In der Wirbelsäulenchirurgie können manuell Bohrungen und Fräsflächen sauberer hergestellt werden. In der Knieendoprothetik können manuell die Schnittflächen sauberer gelegt werden. Es gibt fast in allen Bereichen der Medizin deutliche Vorteile bei der Instrumentenführung. Das Verfahren erlaubt es, auch mit nichttaktilen gewebeabtragenden Effektoren wie beispielsweise Laserstrahlen bei einer manuellen Bearbeitung eines Hartgewebes eine Passform herzustellen, die bestimmten Kriterien (z.B. zylindrisch) genügt.

Ein weiterer Vorteil der Erfindung besteht darin, dass es möglich wird, mit registrierten Werkzeugen unbehindert zu arbeiten, indem die Lage T_HAND mit Position und Orientierung eines Handstücks mit Werkzeugaufnahmevorrichtung erfasst oder berechnet wird, wobei die Übergangsmatrix zwischen Handstück und Werkzeugaufnahme HAND_T_SPANN gespeichert wird und die Übergangsmatrix zwischen Werkzeugaufnahme und Werkzeugeffektor SPANN_T_WERK bis auf einen fehlenden Lage-Freiheitsgrad wie die Länge und ein Registrierpunkt P-REG bekannt ist.

Werkzeuge, die ihre Geometrie im Lauf der Zeit verändern, werden immer wieder eingemessen. Alle Instrumente werden immer eingemessen. Ein Vorteil der Erfindung ist es, dass der Einmessvorgang nicht als Behinderung empfunden wird und keine Taste gedrückt werden muss. Das Einmessen erfordert keinerlei Maschineninteraktion, sondern nur das Berühren eines Punkts und das Abwarten eines Signals. Es kann nicht mehr vergessen werden, ein Werkzeug einzumessen. Die Sicherheit und Bedienbarkeit medizinischer Navigationsyssteme mit wechselnden Werkzeugen steigt erheblich an.

Ein zusätzlicher Vorteil der Erfindung besteht darin, dass herkömmliche Handstücke im Navigationssystem eingesetzt werden können, wobei das Handstück mindestens eine Öffnung zum nachträglichen Befestigen eines Markierungsträgers aufweist und die Öffnung derart ausgebildet ist, dass sie im montierten Zustand von Handstück und Markierungsträger mit einer Erhebung des Markierungsträgers formschlüssig spielfrei in Eingriff steht oder das Handstück mindestens eine Erhebung zum nachträglichen Befestigen eines Markierungsträgers aufweist, wobei die Erhebung derart ausgebildet ist, dass sie im montierten Zustand von Handstück und Markierungsträger mit einer Öffnung des Markierungsträgers formschlüssig spielfrei in Eingriff steht.

Die Erfindung soll nachstehend anhand von zumindest teilweise in den Figuren dargestellten Ausführungsbeispielen näher beschrieben werden.

Es zeigen:
- Fig. 1: Gewebeabtragender Effektor mit Referenzlage zum Gewebeobjekt,
- Fig. 2: Wirkgeometrie und Schnittgeometrie im Objekt,
- Fig. 3: Hergestellter Passkörper und Passform des abgetrage- nen Gewebes,
- Fig. 4: Visualisierung der Differenzgeometrie zur Effektorführung,
- Fig. 5: Drosselintervall zur Steuerung und Regelung der Effektorleistung,
- Fig. 6: Laserhandstück mit passformerzeugender Effektorgeo- metriekörper,
- Fig. 7: Das Setzen von Schnitten in Weichgewebe,
- Fig. 8: Ein System zur Durchführung des manuellen optimalen Gewebeabtrags.
- Fig. 9: Ein Instrumentenhandstück mit Handstückmarkierung
- Fig. 10: Instrumentenhandstück mit Handstückmarkierung,
- Fig. 11: Ausführungsform der Verspannung von markierungsträ- ger und Handstück,
- Fig. 12: Ausführungsform der sicheren Lagefixierung von markierungsträger und Handstückkonus ,
- Fig. 13: Ausführungsform des Handstückmarkierungsträger,
- Fig. 14: Handstück mit Markierungsträger und Markierung

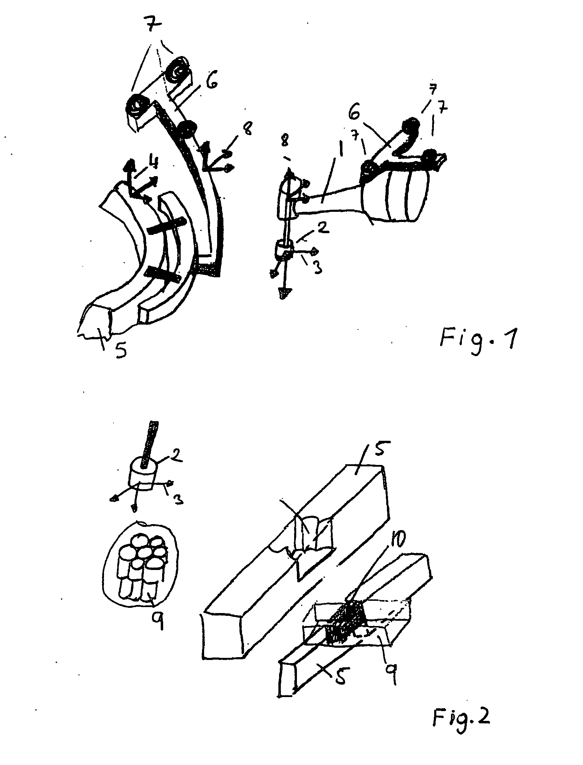

Fig. 1 zeigt das Handstück 1 eines medizinischen Instruments mit einem gewebeabtragenden Effektor 2 in einer messbaren Effektorlage (Position und Orientierung) 3 relativ zu einer Referenzlage 4 eines Gewebeobjekts 5. Die gewebeabtragende Geometrie des Effektors 2 ist als quasi unveränderlich bekannt (z.B. Fräser, Bohrer) oder kann vermessen bzw. justiert werden (z.B. Laser). Die Leistung zum Abtragen des Gewebes kann zumindest ein- und ausgeschaltet werden bzw. vorzugsweise gesteuert oder geregelt werden. Bei dem Effektor 2 kann es sich um ein Sägeblatt, einen Bohrer, eine Fräse, einen Wasser- oder Partikelstrahl, Laserstrahl, Ultraschall oder andere Effektoren zum Gewebeabtragen handeln. Die relative Lage T_EFF des gewebeabtragenden Effektors 3 zur Referenzlage 4 T_OBJ des Gewebeobjekts 5 kann beispielsweise durch Koordinatenmessverfahren ermittelt werden, die auf künstlichen oder anatomischen Messmarkierungen in bekannter Lage aufbauen. In der Fig.1 sind Markierungsträger 6 dargestellt, die fest in ihrer Lage relativ zu Effektor bzw. Gewebeobjekt befestigt sind.

An den Markierungsträgern 6 befindet sich wie in der Figur 1 dargestellt eine Markierung 7 aus reflektierenden Glaskugeln, die als Signalreflektoren in einem optischen Koordinatenmeßsystem zum Einsatz kommen können.

Die Markierung 7 ist verallgemeinert eine Menge von Punkten, Figuren oder Körpern, deren relative Lage (Position und/oder Orientierung) zueinander sowie zu dem jeweiligen Markierungsreferenzsystem 8 vorab bekannt ist und deren Lage relativ zu mindestens einem Lagemeßkoordiantensystem bei Bedarf bestimmt werden kann. Dazu können unterschiedliche Meßverfahren (optisch, akustisch, elektromagnetisch, radarbasiert, laserbasiert, Zeilenkamera, Flächenkameras, Videosequenzen, 3D-Oberflächenkameras, 3D-Laserkameras, 3D-Radarverfahren usw. mit signalsendenden, signalempfangenden und signalreflektierenden Punkten, Figuren oder Körpern) verwendet werden.

Alternativ kann die Markierung 7 als Flansch zur Aufnahme eines Meßfühlers in bekannter Lage relativ zu dem jeweiligen Markierungsreferenzsystem 8 realisiert sein. Die Markierung 7 kann an dem jeweiligen Markierungsträger 6 angebracht, ausgespart und/oder durch einen Teil der Geometrie des Markierungsträgers 6 ausgeprägt sein. Der jeweilige Markierungsträger 6 kann auch durch das Handstück 1 des Effektors 2 oder das Objekt selbst gebildet werden.

Fig. 2 zeigt ein Wirkvolumen bzw. eine Wirkgeometrie 9, die durch die räumliche Überlagerung der Effektorgeometrie 2 an den gemessenen Effektorlagen 3 berechnet wird. Das Wirkvolumen beschreibt die mit dem Wirkeffektor maximal überstrichene Raumgeometrie. Ebenfalls eingezeichnet ist die Schnittgeometrie 10 bzw. das Schnittvolumen, das aus der Schnittmenge von dem Gewebeobjektvolumen 5 in der Referenzlage 4 - vor dem Gewebeabtrag - und dem Wirkvolumen 9 gebildet wird. Das Schnittvolumen beschreibt das mit dem Effektor 2 tatsächliche abgetragene Objektvolumen. Die für den Gewebeabtrag relevante Objektgewebegeometrie kann über ein tiefenbild- bzw. volumenbilderzeugendes Verfahren (Röntgen, Ultraschall, Laser, MRT, CT, - oder Oberflächenbild etc.) oder über ein oberflächenbilderzeugendes Verfahren (2D, 3D Oberflächenscanner, Videobild, Handscanner) oder über ein taktiles oder nichttaktiles abstandsbilderzeugendes Verfahren (Abstandslaser, taktiler Messfühler etc.) mit nachfolgender Oberflächennetzgenerierung erzeugt werden. Im einfachsten Fall wird mit der energielosen Effektorgeometrie 2 die Oberfläche berührt und so die Oberfläche taktile vermessen (durch Erzeugung eines Oberflächengitters aus den Messpunkten) oder am Handstück 1 ist ein abstandsmessender oder oberflächenmessender Sensor befestigt bzw. in das Handstück 1 integriert.

Das Schnittvolumen 10 wäre in der Zahnheilkunde beispielsweise eine gebohrte, gefräste oder gelaserte Kavität im Zahn, im Kieferknochen oder in einem Modell. Das Schnittvolumen 10 kann in der Zahnheilkunde auch das Gewebe beschreiben, das abgetragen wurde, um einen Stumpf für eine Überkronung herzustellen. Entsprechende Beispiele gibt es in der Chirurgie. Das Schnittvolumen 10 kann auch eine Schnittfläche zum Trennen von Gewebe in der Hartgewebschirurgie (Osteotomie) oder aber auch eine Schnittfläche in der Weichgebschirurgie (z.B. Viszeralchirurgie) beschreiben.

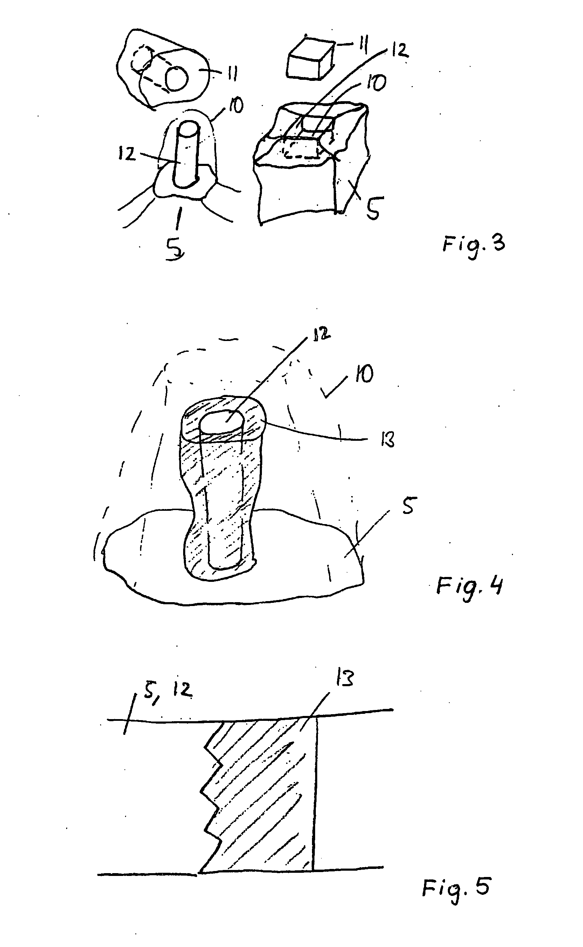

Fig. 3 zeigt Gewebeobjekte 5 mit abgetragenem Gewebevolumen 10 sowie die Geometrie von Passkörpern 11, die auf der Basis der Schnittvolumengeometrie 10 sowie weiteren medizinischen Kriterien und/oder Kriterien zur Herstellung der Passkörper 11 und/oder Integration von Passkörper 11 und Restgewebevolumen 5,12 beruhen. Medizinische Kriterien können sein, dass beispielsweise die Außen- oder Innenoberfläche des Passkörpers 11 einen Mindestabstand zu dem abgetragenen Gewebe 10 oder zu Gewebe mit bestimmten Gewebeeigenschaften (tumorös, bakteriell, Hartgewebe, Spongiosa, Außenschale, Nerven, Organe etc.) besitzt oder es beispielsweise keine Fallen (Hohlräume) für Bakterien geben darf. In der Zahnheilkunde muss der Passkörper 11 auch weiteren medizinischen Kriterien wie der optimalen Okklusion (Passung zwischen den Zähnen unterschiedlicher Kiefer) genügen. Kriterien zur Herstellung der Passkörper 11 können sein, dass Grundkörper oder Materialmengen in einem Lager vorhanden sind, oder sich die Passkörper 11 mit bekannten und/oder vorhandenen Werkzeugen bzw. Werkzeugmaschinen herstellen lassen, und diese daher bestimmte Materialeigenschaften (z.B. Festigkeit oder besondere Geometrieformen) aufweisen müssen. Ein Kriterium kann auch sein, dass der entsprechende Passkörper 11 in einem Lager vorhanden sein muss.

Kriterien zur Integration von Passkörper 11 und Restgewebevolumen 5,12 können sich auf die Fügepassung zwischen Passkörper 11 und Restgewebevolumen, d.h. Objektpassform 12 beziehen, da Hartgewebe so passend vorbereitet sein muss, dass sich der Passkörper 11 sauber fügen lässt. Dies fordert auch bestimmte geometrische Formen. Auch die Vergrößerung und oder die Verkleinerung der Passkörpergeometrie, so dass eine gewünschte Endform nach dem Verbinden von Restgewebevolumen und Passkörper 11 entsteht, fällt hierunter.

Die Passkörpergeometrie kann jetzt dazu verwendet werden, beispielsweise eine Materialmenge oder ein Materialvolumen abzumessen oder die Daten zur Herstellung des Passkörpers 11 mit Hilfe von CAD/CAM-Verfahren bzw. im Rapid-Prototyping-Verfahren zu verwenden. So kann beispielsweise eine Fräsmaschine angesteuert werden, die den Passkörper 11 aus einem Grundkörper heraus fräst.

Alternativ dazu kann auch ein passender Grundkörper aus einem Lager ausgewählt und entnommen werden, der nicht oder nur wenig geeignet nachbearbeitet werden muss.

Fig. 4 zeigt ein Gewebeobjekt 5 (Zahnstumpf) bei dem bereits die optimale Objektpassform 12 durch den Passkörper 11 bekannt ist, jedoch noch nicht alles abzutragende Gewebe abgetragen wurde. Im Bild ist das Differenzvolumen 13 zu erkennen, das aus der Geometrie des aktuellen Passkörpers 12 und dem aktuellen Schnittvolumen 10 durch Schnittbildung ermittelt wird. Das Differenzvolumen 13 bzw. die Differenzgeometrie kann an einem Bildschirm visualisiert werden und/oder es kann akustisch der Abstand des Effektors 2 zur Grenzfläche Differenzvolumen 13 und Passform 12 signalisiert werden. Die Visualisierung wird dann dazu verwendet, manuell (Hand-Auge und/oder Hand-Ohr-Koordiantion) oder motorisch angetrieben und geregelt (z.B. mit einem Roboter) den Effektor 2 so zu bewegen, dass der Effektor 2 nur Gewebe des Differenzvolumens 13 erreichen soll oder kann. Dadurch wird der Gewebeabtrag optimal minimiert. Da kontinuierlich die Schnittgeometrie ermittelt wird kann das System auch zur Vermessung und Dokumentation selbsterzeugter Kavitäten sowie zur Weiterverarbeitung der Messdaten verwendet werden. Der Effektor 2 kann auch als taktiler Positionsmesstastkopf zum Einsatz kommen.

Fig. 5 zeigt das Gewebeobjekt 5 bzw. die Objektpassform 12 sowie die Differenzgeometrie 13, die das noch abzutragende Gewebe beschreibt. Die Leistung des gewebeabtragenden Effektors 2 wird spätesten dann abgeschaltet, wenn die Effektorgeometrie die Differenzgeometrie 13 oder die Vereinigungsmenge von Passkörpergeometrie 11 und Differenzgeometrie 13 verlässt. Die Leistung des gewebeabtragenden Effektors 2 wird spätesten dann eingeschaltet, wenn sich die Effektorgeometrie in die Differenzgeometrie 13 oder die Vereinigungsmenge von Passkörpergeometrie 11 und Differenzgeometrie 13 hineinbewegt. Die Leistung des Effektors 2 wird in Abhängigkeit von dem Abstand des Effektors 2 zur Objektpassform 12 gesteigert bzw. mit sinkenden Abstand reduziert. Vorzugsweise ist die Leistungsveränderung auf ein Drosselintervall ausgehend von der Objektpassformoberfläche 12 beschränkt.

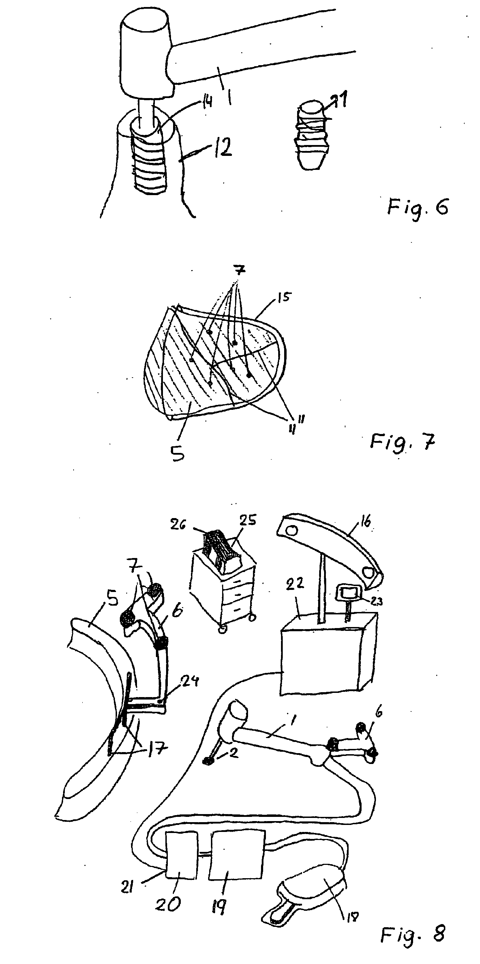

Fig. 6 zeigt ein Handstück 1 (z.B. Laserhandstück) mit einem aufgesetzten Effektorgeometriekörper 14, welcher die gewebeabtragende Energie so kontrolliert an der Grenzfläche abgibt, dass sich eine gewünschte Passform 12 für einen Passkörper 11 ergibt. Bei einem Lasereinsatz und einem geeigneten lichtleitenden bzw. lichtemittierenden Effektorgeometriekörper 14 kann dann beispielsweise ein Außen- oder Innengewinde mit dem Laser so geschnitten werden, dass es sich an der exakten gewünschten Lage (Position und Orientierung) befindet und darüber hinaus sogar der Endpunkt der austretenden Windung bekannt sein kann.

Fig. 7 zeigt ein Weichgewebeobjekt 5, in das zwei Schnitte II gesetzt werden sollen, die in diesem Fall als Passkörper modelliert sind. Die Lage des Gewebes wird über Markierungen 7 erfasst, die beispielsweise mit einem elektromagnetischen Positionsmessverfahren, ähnlich einem GPS vermessen werden. Mit diesem Verfahren können auch Teilvolumen des Weichgewebes in ihrer Position und Lage bestimmt werden. Das Gewebe befindet sich vorzugsweise in einer formstabilisierenden Matrize 15, damit sich das Gewebe 5 bei dem Trennvorgang nicht verschiebt. Die formstabilisierende Matrize 15 sollte ihre Form auch beim Setzen der Schnitte behalten können. Diese könnte bei einem Skalpelleffektor 2 eine Folie sein oder ein Körper mit vorgefertigten vorzugsweise gitterförmigen oder bei einem Laserskalpell eine lichtdurchlässige, lichtleitende Folie sein die die Laserleistung auf der Gewebeseite des Matrize 15 gewebeabtragend austreten lässt.

Fig. 8 zeigt in Zusammenschau mit Fig.1 ein erfindungsgemäßes Gerätesystem bestehend aus einem Lagemeßsystem 16 zur Messung der Effektorlage 3 eines Effektors 2 in einem Handstück 1 relativ zu der Referenzlage 4 eines Gewebeobjekts 5. In Fig. 8 ist das Lagemeßsystem als optisches Navigationssystem 16 dargestellt, mit als kugelförmige passive Marker ausgebildeten Markierungen 7, deren Träger 6 über provisorische Implantate 17 mit dem Kieferknochen verschraubt sind bzw. an dem Handstück 1 angebracht sind.

Die über ein Leistungsteuergerät 18 (Fußschalter, Handschalter, Sensor) kontrollierte Leistung des Leistungsumsetzers 19 (Antriebsmotor) für den material- oder gewebeabtragenden Effektor 2 kann über eine Drosselvorrichtung 20 ausgeschaltet und/oder eingeschaltet und/oder auf eine geeignete Leistung reduziert werden. Die Drosselvorrichtung 20 kann auch integraler Bestandteil des Leistungsumsetzers 19 sein und über eine Gerätedrosselschnittstelle 21 angesteuert werden.

Eine Steuereinheit 22, vorzugsweise ein Computer mit Display 23 (z.B. Bildschirm mit Lautsprecher), wird verwendet, um die Messdaten des Lagemeßsystems 16 einzulesen und auszuwerten.

Bei Bedarf wird zu Beginn die Länge oder die Form des Effektors 2 in einer Einmessvorrichtung 24, im vorliegenden Ausführungsbeispiel Registrierpunkt ausgebildet, eingemessen. In der Steuereinheit ist nach definierten Kriterien mindestens eine Lage (Position und Orientierung) des abzutragenden Materials oder Gewebes 5 bekannt oder kann im Betrieb (on-line) festgelegt werden. Es ist mindestens eine Passform 12 und/oder ein Passkörper 11 vorab gespeichert oder kann im Betrieb (on-line) definiert werden. Alternativ dazu oder zusätzlich sind Kriterien zur on-line dynamischen Berechnung von mindestens einer Passform/ Passkörper 11,12 vorab gespeichert oder die entsprechenden Kriterien können im Betrieb (on-line) festgelegt werden. In der Steuereinheit ist während des Betriebs eine Objektgeometrie 5 gespeichert, die entweder vorab bekannt war, vor dem Einsatz vermessen wurde oder eingriffsbegleitend kurz vor dem Gewebeabtrag vermessen wird. Die Steuereinheit berechnet jetzt bei Bedarf oder quasikontinuierlich das Wirkvolumen 9, das Schnittvolumen 10, wählt oder berechnet die Passkörpergeometrie 11 und die geeignete Passform 12 und berechnet die Differenzgeometrie 13. Die Differenzgeometrie wird geeignet auf dem Bildschirm dargestellt und erlaubt das manuelle Anordnen und Führen des Handstücks so, dass gezielt die Differenzgeometrie abgetragen werden kann. Dabei kann die Steuereinheit 22 die Leistung des Effektors 2 über die Drosselschnittstelle 21 aus- und einschalten bzw. drosseln wie weiter oben beschrieben. Anschließend wird aus einem Lager 25 der bereits vorbereitete geeignete Passkörper entnommen (beispielsweise vom Zahntechniker oder als Normkörper von einer Dentalfirma hergestellt) oder nachträglich hergestellt (vom Zahntechniker oder von einer Fertigungsmaschine) . Der Körper wird mit der Passform integriert und geeignet nachbearbeitet, wobei die Leistungsdrosselung dann ständig freigeschaltet werden kann. Wird ein Modell bearbeitet, dann kann dieselbe Bearbeitung an einem anderen Modell oder Patientengewebe kopiert werden.

Zur Herstellung von Kavitäten im Hartgewebe für die Implantologie wird das Verfahren und ein entsprechendes Gerätesystem wie folgt eingesetzt.

An dem Hartgewebe 5 wird eine Messmarkierung 7 befestigt, die es erlaubt, die Lage der Hartgewebegeometrie relativ zu einem Referenzkoordinatensystem 8 quasikontinuierlich zu bestimmen oder zu messen. Das Hartgewebe 5 kann dabei ortsfest fixiert oder frei beweglich sein. Die relative Lage des Hartgewebes zu den Messmarkierungen kann mit unterschiedlichen abstands-, volumen-, oder oberflächenbildgebende Verfahren ermittelt werden. In der Zahnheilkunde und der Kopfchirurgie bietet sich die Verwendung einer an den Zähnen befestigten Registratschablone an, in anderen Gebieten der Chirurgie eine Oberflächenvermessung oder eine Markerregistrierung.

Der Effektor 2 kann beispielsweise eine Fräse, ein Bohrer oder ein Laser sein, der über ein entsprechendes Handstück 1 manuell (aber natürlich auch kinematisch gestützt, gebremst, gedämpft oder angetrieben) geführt wird. Am Handstück 1 ist ebenfalls eine Messmarkierung 7 vorhanden. Ein Positions- und/oder Lagemesssystem 16 wird verwendet, das es erlaubt, die relative Lage der Marker und damit auch die Markierungsreferenzsysteme 8 zu vermessen. Hier können optische, elektromagnetische, akustische, oberflächenabstandsmessende, Navigationssysteme mit festen oder variablen Markergeometrien zum Einsatz kommen. Besonders einfach ist in der Zahnheilkunde die Verwendung von optischen Navigationssystemen mit passiven Markern. Die Geometrie und die Lage des Effektors 2 relativ zu dem Markierungsreferenzsystem 8 des Handstücks 1 ist vorab bekannt, oder wird durch das Berühren eines Registrierpunkts 24 bzw. einer Registrierform eingemessen. Bei einem Laserhandstück ist es auch möglich, den Fokuspunkt entsprechend einzustellen oder seine Position zu vermessen. So kann über eine Koordinatentransformation die Lage 3 der gewebeabtragenden Effektorgeometrie mit dem Navigationssystemen relativ zu der Lage 4 des Objektgewebes 5 quasikontinuierlich gemessen werden. Manuell oder mit Hilfe einer Kinematik wird mit dem Effektor 2 jetzt Gewebe abgetragen, wobei - vorzugsweise mit einem Computer - die Positionen und Orientierungen der gewebeabtragenden Effektorgeometrie protokolliert werden und aus der Überlagerung der Effektorgeometrien eine Wirkgeometrie 9 und aus der Schnittbildung von Objektgeometrie 5 und Wirkgeometrie 9 die Geometrie des abgetragenen Gewebevolumens 10 berechnet wird. Es wird also direkt die Geometrie des abgetragenen Gewebes berechnet. Beim Abtragen wird der Arzt versuchen, bestimmte Kriterien einzuhalten. Dies können Informationen über das Gewebe sein, die beispielsweise über die Augen (Farbe, Späne), Nase (Gerüche), die taktile Informationen (Gewebefestigkeit oder Festigkeitsänderung des Gewebe) oder akustisch wahrgenommen werden und direkt umgesetzt werden. Es können auch Informationen aus einer Vorplanung sein, bei der bestimmte Positionen, Orientierungen der Kavitäten oder Kavitätsgeometrien festgelegt wurden. Dabei kann die Kavität beispielsweise zur Aufnahme eines Implantats geformt werden müssen. Im einfachsten Fall wird die Kavität für ein vorab definiertes Implantat oder Transplantat angefertigt. Es ist aber auch möglich, dass unter Berücksichtigung weiterer Kriterien ein Implantat aus einer Auswahl verschiedener vorhandener Implantate ausgewählt wird. In diesem Fall muss die Kavität jetzt auch noch den Kriterien einer Passform 12 für den Passkörper 11 des Implantats genügen. Aus diesem Grund wird ein Differenzkörper 13 berechnet, der das Gewebe umschließt, das noch entfernt werden muss, um die Passform 12 für den Passkörper 11 zu bilden. Diese Differenzgeometrie 13 wird dazu verwendet, um den Effektor 2 für den Gewebeabtrag optimal anzuordnen und zu führen. Die kann beispielsweise durch eine grafische Darstellung an einem Bildschirm für den Arzt erfolgen oder durch die Steuerung einer roboterähnlichen Kinematik. Auf der Basis der Differenzgeometrie kann auch ein Effektorgeometriekörper 14 ausgewählt werden, der eine Passform 12 direkt erzeugt. Dies kann beispielsweise bei einem Laserhandstück mit einem gewebeabtragenden Laser mittels eines zylinderförmigen Effektorgeometriekörpers 14 geschehen, der so lichtdurchlässig ist, dass das gewebeabtragende Laserlicht beim Austritt aus dem Effektorgeometriekörper 14 ein Gewinde als Passform 12 in das Hartgewebe 5 schneidet. Ist die Passform 12 im Gewebe 5 hergestellt, dann kann das Implantat bzw. der Passkörper 11 aus einem Lager 25 entnommen werden und direkt integriert werden. Um zu vermeiden, dass bei der manuellen oder kinematisch gestützten Anordnung und Führung des Effektor 2 versehentlich Gewebe 5 abgetragen wird, dass die optimale Passform 12 zerstören würde oder den erforderlichen Kriterien nicht genügt, wird die gewebeabtragende Effektorleistung computergesteuert abgeschaltet, wenn sich der Effektor 2 außerhalb der Differenzgeometrie 13 und/oder einer Teilmenge der Vereinigungsmenge von Differenzgeometrie 13 und Passkörpergeometrie 11 befindet. Vorzugsweise wird die Effektorleistung aus Sicherheitsgründen auch nur eingeschaltet, wenn sich der Effektor 2 innerhalb der Differenzgeometrie 13 und/oder einer Teilmenge der Vereinigungsmenge von Differenzgeometrie 13 und Passkörpergeometrie 11 befindet.

Um eine besonders saubere Passform 12 zu erhalten ist es sinnvoll, die Effektorleistung mit sinkendem Abstand des Effektors 2 zur Grenzfläche zwischen Passform12 und Differenzgeometrie 13 so zu drosseln, dass möglichst kein Gewebe der Passform 12 versehentlich abgetragen werden kann. An dem Handstück 1 ist vorzugsweise ein Absaugmechanismus für das Absaugen von Gerüchen sowie Dämpfen und anderen Partikeln angebracht.

Zur Herstellung von Kavitäten im Zahn für die Versorgung mit Inlays, Onlays oder Überkronungen wird das Verfahren und ein entsprechendes System analog zu der Bearbeitung am Knochen eingesetzt. Hier wird jedoch der Passkörper 11 in Form eins Inlays, Onlays oder einer Brücke entweder aus einem Lager mit vorbereiteten Normkörper entnommen oder im Rapid-PrototypingVerfahren (gefräst, gesintert, usw.) oder vorab von einem Zahntechniker hergestellt und vermessen. Es kann auch Material abgemessen und in die Kavität bzw. in eine Form um den Passkörper herum eingefüllt werden.

Das Verfahren kann in der Zahnmedizin auch für die Herstellung oder Modifikation von Modellarbeiten und Suprakonstruktionen verwendet werden. Dann wird nicht nur am Patientengewebe, sondern auch an Modellen bzw. an den Suprakonstruktionen gearbeitet, die sich jedoch über bekannte Verfahren (Registrierschablone übertragen lassen).

Das Verfahren kann auch in der Kniendoprothetik eingesetzt werden, bei der sehr viele Schnitte am Knochen gesetzt bzw. Flächen zueinander gefräst werden müssen. Hier kann sehr einfach ein Markierungsträger an den Knochen angeschraubt werden.

Das Verfahren kann auch in der Dekompression und der Vorbereitung der Verschraubung von Wirbelkörpern verwendet werden.

Zum Trennen von Hartgewebe wird das Verfahren und ein entsprechendes System wie folgt eingesetzt. Der Passkörper wird als mindestens eine Schnittfläche definiert bzw. als mindestens ein Schnittvolumen. Es muß nicht notwendigerweise auch ein Passkörper eingesetzt werden.

Zum Trennen von Weichgewebe beispielsweise in der Viszeralchirurgie wird das Verfahren und ein entsprechendes System wie folgt eingesetzt. Es wird verwendet, um saubere Schnitte in Weichgewebe 5 zu setzen, um beispielsweise Gewebe zu trennen oder zu entfernen. Dabei wird die Lage (Position und Orientierung) 4 des Weichgewebes 5 beispielsweise über ein Weichteil-GPS verwendet, bei denen in das Weichgewebe Markierungen 7 eingebracht sind. Über die Lagemessung der Markierungen kann die teilweise unabhängige Verschiebung und Verlagerung von Gewebestrukturen vermessen werden. Zusammen mit einem leistungsgesteuerten Effektor 2 zur Gewebetrennung lassen sich dann saubere Schnitte setzen. Das Gewebe kann auch vorab in eine formstabilisierende Matrize 15 gelegt, gepresst oder gesaugt werden, bevor die gewebeabtragende Leistung zugeführt wird. Die Matrize 15 kann auch selbst energiedurchlässig sein, damit die Schnitte geeignet durch die Matrize hindurch geführt werden können. Das Schneidwerkzeug ist hier vorzugweise ein Laser, der abtastend über das Gewebe geführt und selbständig die Schneidposition misst. Die Leistung wird nur an den geplanten Schnittkanten bzw. Schnittflächen zugeschaltet. Die formgebende Matrize 15 kann dabei aus einem lichtleitenden Material sein.

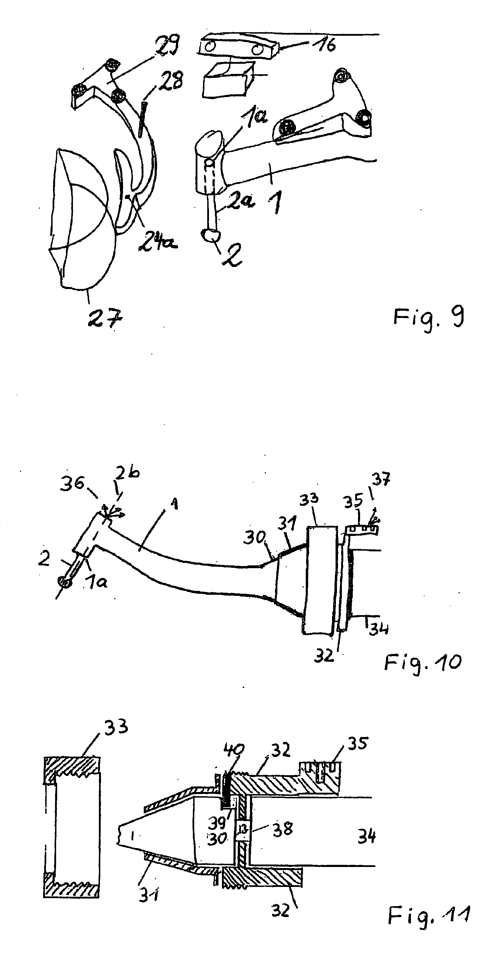

Der Vorgang des automatisierten Einmessens wird an Hand von Fig. 9 näher erläutert.

Fig. 9 zeigt ein Handstück (1) mit einer Werkzeugaufnahmevorrichtung (1a) und einem eingespannten Werkzeug (2a), für das die Lage (Position und Orientierung) des Werkzeugeffektors (2) gemessen werden soll. Zu sehen ist ebenfalls der Registrierpunkt (24a), das Arbeitsvolumen (27) und der Kalibrationkörper (28), der im vorliegenden Ausführungsbeispiel als Stift ausgelegt ist. Die Positionsmessung wird von dem Positionsmeßsystem (16) durchgeführt, das auch die Übergangsmatrizen relativ zu einem Referenzkoordinatensystem (29) speichert.

Im Bild ist es als optisches Navigationssystem mit passiven Reflektoren dargestellt.

Nach dem Einschalten des Systems wird dem Benutzer signalisiert, das Handstück (1) zu kalibrieren. Dazu steckt er es auf den Passkörper. Jetzt kann er ein Werkzeug (2a), z.B. einen Bohrer, einspannen. Bevor er in das Arbeitsvolumen (27 eindringt berührt er mit der Werkzeugspitze den Registrierpunkt (24a) und wartet das Registriersignal ab. Danach arbeitet er navigiert im Arbeitsvolumen (27). Legt er das Instrument außerhalb des Arbeitsvolumens (27) ab, dann muss er beim nächsten Eindringen in das Arbeitsvolumen (27) erneut den Registrierpunkt (24a) berühren. Gleiches gilt für die Situation nach einem Werkzeugwechsel.

Fig. 10 zeigt ein Handstück 1 mit einer Effektoraufnahme 1a, wie beispielsweise ein Spannfutter, zum Einspannen eines Effektors 2, wie beispielsweise ein Bohrer.

Die Effektoraufnahme 1a besitzt eine Effektorreferenzlage 36, die Ursprungsposition und die Ursprungsorientierung eines Effektors 2, der sich in der Effektoraufnahme 1a befindet, definiert.

An dem Handstück 1 kann ein Handstückmarkierungsträger 32 mit einer Handstückmarkierung 35 so befestigt werden, dass die Handstückmarkierung 35 in mindestens einer vorab bekannten Lage (Position und Orientierung) relativ zu der Effektorreferenzlage 36 angebracht und fixiert werden kann.

Der Handstückmarkierungsträger 32 mit der Handstückmarkierung 35 kann entweder dauerhaft oder abnehmbar an dem Handstück 1 fixiert werden. Das Handstück 1 kann auch selbst der Handstückmarkierungsträger 32 sein.

Fig. 11 zeigt eine Form der oben beschriebenen Fixierung des Handstückmarkierungsträgers 32 an dem Handstück 1. Über den Handstückkonus 30 ist ein hohlkegelstumpfförmiger Überwurfkonus 31 geschoben. Er dient als Gegenlager für die Verspannung des Handstückmarkierungsträgers 32 mit dem Handstück 1. Der Innendurchmesser des Überwurfkonus 31 ist ausreichen groß, um über die Effektoraufnahme 1a bis zum Handstückkonus 30 geschoben werden zu können, jedoch kleiner als der Außendurchmesser des Handstückkonus 30 an seinem größten Umfang. Die Fixierung von Handstückmarkierungsträger 32 am Handstück 1 wird mit einer Überwurfmutter 33 erreicht, deren Innendurchmesser ausreichen groß ist, um über Effektoraufnahme 1a und Handstück 1 geschoben werden zu können, jedoch kleiner ist als der Außendurchmesser des Überwurfkonus 31 an seinem größten Umfang. Das Innengewinde der Überwurfmutter 33 wird auf das Außengewinde des Markierungsträgers 32 geschraubt.

Der Handstückmarkierungsträger 32, Überwurfkonus 31 und Überwurfmutter 33 sind vorzugsweise aus leichtem, formstabilen und sterilisierbarem Material hergestellt. Alle drei Teile behindern weder den Anschluss des Effektorantriebs 34 über die Kupplung 38 noch die manuelle Nutzung des Handstücks 1. Die Überwurfmutter 33 hat auf der Außenseite eine aufgeraute Oberfläche, damit sie sich besser von Hand aufschrauben lässt.

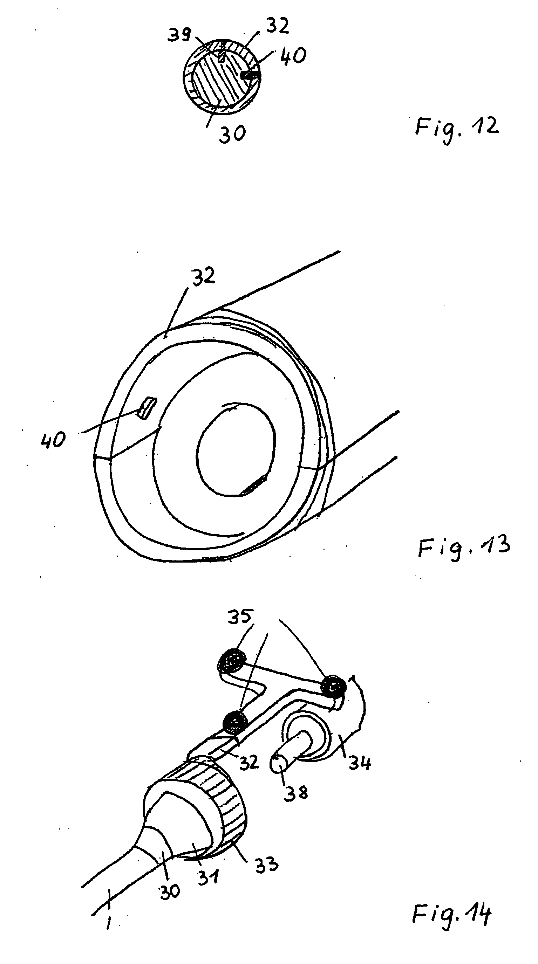

Fig. 12 zeigt einen Handstückkonus 30, der mindestens eine Nut als Aussparung ausgebildete Öffnung 39 zum Einschieben von mindestens einer als Registrierungsfeder ausgebildeten Erhebung 40, die seitlich am Handstückmarkierungsträger 32 angebracht ist, besitzt. Eine Ausführungsform ist das Einstecken einer als Stift ausgebildeten Erhebung 40 durch eine seitlich in das Außengewinde des Markierungsträger eingebrachte Bohrung. Stift 40 und Aussparung 39 müssen als Passung ausgelegt sein, um ein Verdrehen des Markierungsträgers gegenüber der Effektorreferenzlage 37 zu verhindern.

Um ein Winkelhandstück 1 in seiner Ausrichtung für Oberkiefer und Unterkiefer optimal markieren zu können, sollten die als Nuten ausgebildeten Öffnungen 39 ein zweites Mal um 180 Grad um die Antriebsachse rotiert eingebracht werden. Anstelle des Stiftes 40 im Markierungsträger 32 und der Nut 39 im Konus 30 kann auch der Stift 40 im Konus 30 und die Nut 39 im Träger 32 eingebracht sein.

Fig. 13 zeigt eine Ausführungsform des Handstückmarkierungsträgers 32, der aus zwei Hälften besteht, die zusammen gesteckt dieselbe Geometrie wie in Figur 11 ergeben. In dieser Ausführungsform kann sich die Aussparung 39 im Handstück 1 auf eine flache Bohrung beschränken, da die beiden Hälften aufgesteckt werden können, und dabei der Stift 40 direkt passend in die Bohrung 39 gelangt. Es ist vorteilhaft die beiden Hälften als Steckverbindung zu gestalten. Da die Überwurfmutter 33 die beiden Hälften um das Handstück herum fixiert, kann bei dieser Lösungsvariante auf den Überwurfkonus 31 verzichtet werden.

Fig. 14 zeigt ein Handstück 1 mit einem Markierungsträger 32, der durch Überwurfkonus 31 und Überwurfmutter 33 fest mit dem Handstück 1 in vorab bekannter Lage verbunden ist. An dem Markierungsträger 32 befindet sich eine Markierung 35 aus reflektierenden Glaskugeln, die als Signalreflektoren in einem optischen Koordinatenmeßsystem zum Einsatz kommen können.

Die Markierung 35 ist verallgemeinert eine Menge von Punkten, Figuren oder Körpern, deren relative Lage (Position und/oder Orientierung) zueinander sowie zu einem mehrdimensionalen Lagereferenzkoordinatensystem 37 vorab bekannt sind und deren Lage relativ zu mindestens einem Lagemeßkoordinatensystem bei Bedarf bestimmt werden kann. Dazu können unterschiedliche Messverfahren (optisch, akustisch, elektromagnetisch, Radar, Laser, Zeilenkamera, Flächenkameras, Videosequenzen, 3D-Oberflächenkameras, 3D-Laserkameras, 3D-Radarvefahren usw. mit signalsendenden, signalempfangenden und signalreflektierenden Punkten, Figuren oder Körpern) verwendet werden.

Alternativ kann die Markierung als Flansch zur Aufnahme eines Messfühlers in bekannter Handstückreferenzlage 37 realisiert sein. Die Handstückreferenzlage 37 (Position und Orientierung) der Handstückmarkierung 35 und damit auch der Effektorreferenzlage 36 kann mit mindestens einem Lagemeßsystem relativ zu dem Referenzkoordinatensystem des jeweiligen Lagemeßsystem bestimmt werden.

Die Handstückmarkierung 35 kann an dem Handstückmarkierungsträger 32 angebracht, ausgespart und/oder durch einen Teil der Geometrie des Handstückmarkierungsträger 32 ausgeprägt sein.

Zuerst wird das Handstück 1 markiert, in dem die Handstückmarkierung 35 angebracht wird. Wird dazu der Überwurfmechanismus verwendet, dann wird der Handstückmarkierungsträger 32 mit dem Handstückkonus 30 über die Trägerfeder 40 und die Konusaussparung 39 verstiftet. Anschließend wird der Überwurfkonus 31 und danach die Überwurfmutter 33 über das Handstück geschoben, und der Überwurfkonus 31 mit dem Handstückmarkierungsträger 32 soweit mit der Überwurfmutter 33 verspannt, bis der Endanschlag erreicht ist bzw. bis die Trägerfeder 40 maximal in die Konusaussparung 39 eingesteckt ist. Jetzt kann die Lage des Handstücks 1 über die Handstückmarkierung 35 mit einem Lagemeßsystem vermessen werden. Der Antrieb 34 kann über die Kupplung 38 noch nachträglich angeschlossen werden.

Das Verfahren kann auch außerhalb der Medizin z.B. beim Materialabtrag in Manufakturen oder handwerklichen Kleinbetrieben (Schreiner, Zimmermann, Holzbootsbau) oder von Heimwerkern eingesetzt werden, wo keine vollautomatischen computergesteuerten Bearbeitungsmaschinen eingesetzt werden können. Dies kann der Fall sein, weil die Maschinen beispielsweise zu groß oder zu teuer oder überhaupt nicht zu erwerben sind. In diesem Fall können das Verfahren und eine entsprechend ausgerüstete manuelle Bearbeitungsmaschine (elektrisches Heimwerkerbearbeitungsgerät) ein Ergebnis erbringen, das mit einer numerisch gesteuerten automatischen Maschine vergleichbar ist. Ein Anwendungsbeispiel hier ist das "in Form"-Schleifen zum Restaurieren eines alten Autos oder das Abschleifen einer GFK-Hülle eines alten Segelboots. Hier wird ein Normkörper auf der Basis von alten bekannten Rissen bzw. technischen Zeichnungen vorgegeben. An dem Bearbeitungsobjekt wird der Referenzpunkt einer Koordinatenmesseinrichtung angebracht wie Beispiel der eines Differenz-GPS oder einer optischen bzw. Laser, Radar etc. basierten Koordinatenmesseinrichtung oder eines Messarms. Die Effektorgeometrie beispielsweise der Schleifscheibe ist entweder bekannt oder wird in einer Form eingemessen. Die Effektorposition der gewebeabtragende Effektorgeometrie der Maschine (Schleifmaschine, Fräskopf, Polierkopf) wird kontinuierlich bestimmt und beim Schleifen die Leistung (Drehzahl) der Maschine so definiert, dass die Maschinenleistung als Funktion des Abstands zwischen aktueller Lage des gewebeabtragenden Effektor und der Oberflächenlage der Normgeometrie geregelt wird. So läuft die Maschine beispielsweise mit voller Leistung bis zum einem Abstand von 2 mm zur Oberfläche und wird dann bis zum Abstand von 0 mm zur Oberfläche proportional in der Leistung herunter geregelt. Andere Regelungsverfahren können anwendungsabhängig sinnvoll sein. Die Lage des Normkörpers bzw. der Normgeometrie relativ zur Objektgeometrie kann dadurch erreicht werden, dass die Lage des Objektkörpers beispielsweise über das Berühren von mindestens einer Symmetrieachse (z.B. Bugspitze, Heckecken, Ruderfusspunkt am Kiel) mit einem Positionsmessfühler oder der Oberflächenerfassung und Registrierung einer besonders ausgeprägten Teilgeometrie des Objektkörpers durch Mittelung, Oberflächenvermessung und Symmetriebildung.

Das Verfahren kann auch dazu verwendet werden, bei einer unsauberen und unsymmetrischen gespachtelten Oberfläche nachträglich eine Oberfläche zu erzeugen, die definierten Optimierungskriterien genügt, z.B. geringer Luft- oder Wasserwiderstand oder Symmetrie bei minimalem Materialabtrag etc.

Das Verfahren kann auch dazu verwendet werden, um nachträglich Planken oder Spanten an der optimalen Position einzusetzen, wobei dann der Objektkörper passgenau für einen Passkörper vorbereitet wird oder der Passkörper vorbereitet wird für das Einbringen in den Objektkörper.

Zu bearbeitende Materialien können beispielsweise Metall, Glas, Keramik, Holz, Kunststoff in unterschiedlichen Anwendungsgebieten sein.

Die Erfindung ist nicht beschränkt auf die hier dargestellten Ausführungsbeispiele. Vielmehr ist es möglich, durch Kombination der genannten Mittel und Merkmale weitere Ausführungsvarianten zu realisieren, ohne den Rahmen der Erfindung zu verlassen.

### Bezugszeichenliste

- 1: Handstück
- 1a: Werkzeugaufnahmevorrichtung
- 2: Effektor
- 2a: Werkzeug
- 2b: Effektorachse
- 3: Effektorlage
- 4: Referenzlage
- 5: Gewebeobjekt
- 6: Markierungsträger
- 7: Markierung
- 8: Markierungsreferenzsystem
- 9: Wirkvolumen/-geometrie
- 10: Schnittvolumen/-geometrie
- 11: Passkörpergeometrie
- 12: Objektpassform/-geometrie
- 13: Differenzkörpergeometrie
- 14: Effektorgeometriekörper
- 15: formstabilisierende Matrize
- 16: Lagemeßsystem
- 17: provisorische Implantate
- 18: Leistungssteuergerät
- 19: Leistungsumsetzers
- 20: Drosselvorrichtung
- 21: Drosselschnittstelle
- 22: Steuereinheit
- 23: Bildschirm
- 24: Einmessvorrichtung
- 24a: Registrierpunkt
- 25: Lager
- 26: Fertigungsmaschine
- 27: Arbeitsvolumen
- 28: Kalibrationskörper
- 29: Referentkoordinatensystem
- 30: Handstückkonus
- 31: Überwurfkonus
- 32: Handstückmarkierungsträger
- 33: Überwurfmutter
- 34: Effektorantrieb
- 35: Handstückmarkierung
- 36: Effektorreferenzsystem
- 37: Handstückreferenzsystem
- 38: Antriebskupplung
- 39: Öffnung
- 40: Erhebung

## Patentansprüche

1. Verfahren zum Materialabtrag oder zur Materialbearbeitung mittels mindestens eines manuell geführten Effektors unter Einsatz eines Navigationssystems und unter Nutzung von Computertechnik,
Daten zur Positionier und/oder wobei Orientierung des Effektors und deren Änderungen relativ zur Lage mindestens eines Referenzkörpers erfasst, gespeichert und rechentechnisch verarbeitet werden und Befehle zur Steuerung und/oder Regelung auslösen derart, **dadurch gekennzeichnet, dass** der Effektor in Abhängigkeit von einem vorgegebenen Arbeitsvolumen und/oder Materialabtragsvolumen und/oder Materialrestvolumen in Ein-/Aus-Funktion geschaltet wird und in der Ein-Funktion in seiner Leistung und/oder Parameterisierung gesteuert und/oder geregelt wird und dass
die Leistung des materialabtragenden Effektors (2) spätestens dann abgeschaltet wird, wenn es zu einer überschneidung von abgetragenem Material (10) und zu erzielender Objektform (12) kommt.

2. Verfahren nach Anspruch 1, **dadurch gekennzeichnet, dass** die Lage und Geometrie der erreichten Objektoberfläche erfasst und für weitere Bearbeitungsvorgänge am selben oder anderen Objekten gespeichert wird.

3. Verfahren nach Anspruch 1, **dadurch gekennzeichnet, dass** über einen definierten Zeitraum hinweg quasikontinuierlich die Lage (3) einer materialabtragenden Effektorgeometrie (2) relativ zu der Lage (4) eines Objektes (5) erfasst und daraus die Geometrie (10) des abgetragenen Materials berechnet wird, wobei die Geometrie (10) des abgetragenen Materials durch die Einbeziehung von 3D-Oberflächenbilddaten aus einem 3D-Scanner berechnet wird.

4. Verfahren nach Anspruch 1, **dadurch gekennzeichnet, dass** auf der Basis einer definierten Lage und definierten Geometrie einer zu erzielenden Objektform (12) und/ oder auf der Basis der Lage und Geometrie des bereits abgetragenen Gewebes (10) die Lage und Geometrie eines noch abzutragenden Materials (13) berechnet und zur Anordnung und Führung des Effektors verwendet wird und
dass die Leistung des materialabtragenden Effektors zumindest innerhalb der Geometrie des noch abzutragenden Materials (12) auf der Basis des Abstands der Effektorlage (3) und/oder der Effektorgeometrie (2) zur Grenzfläche zwischen abzutragendem Material (13) und zu erzielender Objektform (12) gesteuert oder geregelt wird.

5. Verfahren nach einem der Ansprüche 1 bis 4, **dadurch gekennzeichnet, dass** die materialabtragende Leistung über eine formgebende energietransportierende Matrize ohne Lageveränderung des Effektors (2) direkt zur Erzielung von Teilen der Objektpassform (12) abgegeben wird und/oder dass die zu erzielende Objektform (12) auf der Basis von medizinischen Kriterien und/oder bereits abgetragenem Material und/oder Kriterien zur Herstellung von Objektform und Passkörper und/oder Kriterien zur Integration von Objektform und Passkörper berechnet wird und dass die Geometrie des Passkörpers zur Abmessung einer Materialmenge und/oder Herstellung eines Passkörpers verwendet wird.

6. Verfahren nach Anspruch 5, **dadurch gekennzeichnet, dass** quasikontinuierlich die Lage (3) einer materialabtragenden Effektorgeometrie (2) erfasst und zusammen mit dem Messzeitpunkt gemeinsam gespeichert wird, wobei die materialabtragende Effektorgeometrie bekannt ist und/oder bei Bedarf oder quasikontinuierlich zusammen mit dem Messzeitpunkt und/oder der Lage des Effektorvolumens gemessen, erfasst und gemeinsam gespeichert wird.

7. Verfahren nach einem der Ansprüche 1 bis 6, **dadurch gekennzeichnet, dass** ein Wirkvolumen bei Bedarf oder quasikontinuierlich aus der räumlichen Überlagerung des Effektorvolumens in den erfassten Lagen berechnet wird und vorzugsweise bei Bedarf oder quasikontinuierlich ein Schnittvolumen aus der Schnittmenge von Wirkvolumens mit einem Objektvolumen gebildet bzw. dessen geometrische Beschreibung berechnet wird, wobei die Lage des Objektvolumens relativ zu dem Wirkvolumen bekannt ist oder gemessen wird und vorzugsweise bei Bedarf oder quasikontinuierlich die Schnittvolumenbeschreibung zur Berechnung der Geometrie eines Passkörpers verwendet wird, wobei die Geometrie des Passkörpers geeignet verändert werden kann, um zusätzlichen medizinischen Kriterien oder Kriterien zur Herstellung oder Integration des Passkörpers zu genügen, wobei im einfachsten Fall die Schnittvolumenbeschreibung der Form des Passkörpers entspricht.

8. Verfahren nach Anspruch 1, **dadurch gekennzeichnet, dass** mindestens eine Tabelle mit mindestens einer geometrischen Beschreibung eines Passkörpers als Normkörper verwendet wird und/oder dass aus einer Normkörpertabelle ein Normkörper auswählt wird, der seinem Optimierungskriterium genügt, wobei im einfachsten Fall der Normkörper dem einzigen Eintrag eines Passkörpers in der Tabelle entspricht und es ein der Tabelle der Normkörper entsprechendes Lager mit bereits vorgefertigten Normkörpern gibt, die nicht oder nur in geringem Umfang nachbearbeitet werden müssen.

9. Verfahren nach einem der voranstehenden Ansprüche, **dadurch gekennzeichnet, dass** das Differenzvolumen zwischen dem ausgewählten oder aktuell berechneten Passkörper und dem aktuellen Schnittvolumen berechnet wird und/oder dass die Effektorenergie abgeschaltet wird, wenn sich der Effektor außerhalb der Geometrie des Passkörpers befindet und/oder dass die Effektorenergie eingeschaltet wird, wenn sich der Effektor innerhalb der Geometrie des Passkörpers befindet.

10. Verfahren nach einem der voranstehenden Ansprüche, **dadurch gekennzeichnet, dass** ein weiches Objekt weitgehend formstabil durch eine Matrize stabilisiert wird, die mindestens eine Möglichkeit zur definierten Durchleitung bzw. Durchführung von materialabtragender Energie oder einem materialabtragenden Effektor hat und dass die Differenzvolumengeometrie zur Anordnung und Führung des Effektors verwendet wird und/oder dass beim Abtrag entstehende Abtragspartikel, Dämpfe und Gerüche abgesaugt werden.

11. Verfahren nach Anspruch 1, **dadurch gekennzeichnet, dass** der Effektor nicht nur zum Materialabtragung, sondern auch zur 3D Oberflächengeometrievermessung verwendet wird.

12. Verfahren nach Anspruch 1, **dadurch gekennzeichnet, dass** der Effektor ein Laser ist und die gewebeabtragende Effektorgeometrie durch einen austauschbaren lichtleitenden Positiveffektorgeometriekörper und/ oder durch einen lichtundurchlässigen Negativeffektorgeometriekörper erreicht wird, mit dem sich beispielsweise hinterschneidende Kavitäten herstellen lassen.

13. Verfahren nach Anspruch 1, **dadurch gekennzeichnet, dass** die Objektgeometrie durch ein volumenbildgebendes Verfahren (Röntgen, MRT, CT, Ultraschall, etc.) oder durch ein oberflächenbildgenerierendes Verfahren (3D-Oberflächenscanner, Handscanner, taktiler Meßfühler) direkt oder über einen Abdruck erfasst und berechnet wird und/oder dass ein erster Material- oder Gewebeabtrag an einem Modell erfolgt und die erfasste Lage und Form des Material- oder Gewebeabtrags für den Abtrag an dem selben Modell, anderen Modellen sowie Patienten oder Gegenständen verwendet wird.

14. Verfahren nach Anspruch 1, **dadurch gekennzeichnet, dass** der Material- oder Gewebeabtrag, erfasst über die wirk-, Schnitt- oder Differenzgeometrie oder einen 3D-Oberflächenscanner, als geometrisches Modell gespeichert wird und bei einem weiteren Material- oder Gewebeabtragsvorgang beim selben oder an anderen Objekten oder Modellen verwendet wird, wobei auch Positiv-Geometrien in Negativ-Geometrien gespiegelt werden können, um beispielsweise passend für Kavitäten, Transplantate herauszutrennen.

15. Verfahren nach einem der voranstehenden Ansprüche, **dadurch gekennzeichnet, dass** auf der Basis der Lage und Geometrie des bereits abgetragenen Materials oder Gewebes (10) sowie einer definierten Lage und definierten Geometrie einer zu erzielenden Objektform (12) die Lage und Geometrie des noch abzutragenden Materials oder Gewebes (13) berechnet, an einem Bildschirm grafisch dargestellt wird und die Informationen über die Lage (3) des Effektors (2) zur Grenzfläche zwischen zu erzielender Objektpassform und noch abzutragenden Material oder Gewebe (13) ebenfalls grafisch und/oder akustisch darstellt wird und zur manuellen und/oder kinematischen Anordnung und Führung des Effektors verwendet wird, wobei die aufbereiteten Informationen zur Ansteuerung einer Anordnungs- und Führungkinematik für den Effektor über eine Schnittstelle ausgebbar sind.

16. Verfahren nach Anspruch 1 **dadurch gekennzeichnet, dass** das Arbeitsvolumen derart definiert ist, dass es bei einem normalen Einsatz des Effektors (2) nicht verlassen werden muss, jedoch zum Werkzeugwechsel typischerweise verlassen wird.

17. Gerätesystem zum Material- oder Gewebeabtrag oder zur Material- oder Gewebebearbeitung mittels mindestens eines manuell geführten Effektors unter Einsatz eines Navigationssystems und unter Nutzung von Computertechnik,
wobei ein erster Markierungsträger (6) mit Markierungen (7) an einem Handstück (1) mit Effektor (2) angeordnet und das Handstück (1) mit einer Steuereinheit (22) verbunden und ein zweiter Markierungsträger (6) mit Markierungen (7) an den Material- oder Gewebeobjekt befestigt ist, die Steuereinheit (22) mit einem Lagemesssystem (16) verbunden ist **dadurch gekennzeichnet daß** die Steuereinheit derart ausgebildet ist, die Leistung des materialabtragenden Effektors (2) in Abhängigkeit von der zu erzielenden Objektform durch die Steuereinheit (22) auf der Basis des Abstands der Effektorlage (3) und/oder der Effektorgeometrie zur Grenzfläche zwischen abzutragendem Material (13) und zur zu erzielenden Objektform zu steuern oder zu regeln
und spätestens dann abzuschalter wenn es zu einer Überschneidung von abgetragenem Material (10) und zu erzielender Objektform (12) kommt.

18. Gerätesystem nach Anspruch 17, **dadurch gekennzeichnet, dass** die Steuereinheit (22) mit einem Bildschirm verbunden ist und zwischen Steuereinheit (22) und Handstück (1) ein Leistungssteuergerät (18), ein Leistungsumsetzer (19) oder eine Drosselvorrichtung (20) mit Drosselschnittstelle (21) angeordnet sind und das der zweite Markierungsträger (6) eine Einmessvorrichtung (24) aufweist.

19. Verwendung des Gerätesystems nach Anspruch 17 zum Gewebeabtrag in der Medizin und Zahnmedizin.

20. Verwendung des Gerätesystems nach Anspruch 17 zum Materialabtrag von Metall, Glas, Keramik, Holz, Kunststoff in unterschiedlichen Anwendungsgebieten und für Modellarbeiten.

## Claims

1. A method for removing material or for working material by means of at least one manually guided effector, using a navigation system and utilizing computer technology, wherein data relating to the position and/or orientation of the effector and their changes relative to the position of at least one reference body are collected, stored and computer-processed and initiate commands for controlling and/or regulating, **characterized in that** the effector is switched into an on/off function according to a predetermined working volume and/or material removal volume and/or material residual volume and, in the on function, is controlled and/or regulated in its power and/or parameterization, and that the power of the material-removing effector (2) is switched off at the latest when there is overlapping between removed material (10) and object shape (12) to be achieved.

2. The method according to claim 1, **characterized in that** the position and geometry of the achieved object surface is detected and stored for further working operations on the same or other objects.

3. The method according to claim 1, **characterized in that** the position (3) of a material-removing effector geometry (2) relative to the position (4) of an object (5) is detected quasi-continuously for a defined period of time and the geometry (10) of the removed material is calculated therefrom, the geometry (10) of the removed material being calculated by including 3D surface image data from a 3D scanner.

4. The method according to claim 1, **characterized in that** the position and geometry of a material yet to remove (13) is calculated on the basis of a defined position and defined geometry of an object shape (12) to be achieved and/or on the basis of the position and geometry of the tissue (10) already removed and used for arranging and guiding the effector, and that the power of the material-removing effector is controlled and/or regulated at least within the geometry of the material (12) yet to remove on the basis of the distance from the effector position (3) and/or effector geometry (2) to the boundary surface between material (13) to be removed and object shape (12) to be achieved.

5. The method according to any of claims 1 to 4, **characterized in that** the material-removing power is delivered via a shaping energy-conveying template without changing the position of the effector (2) to directly furnish portions of the object fitting shape (12) and/or that the object shape (12) to be achieved is calculated on the basis of medical criteria and/or material already removed and/or criteria of furnishing object shape and fitting body and/or criteria for the integration of object shape and fitting body, and that the geometry of the fitting body is used for metering an amount of material and/or producing a fitting body.

6. The method according to claim 5, **characterized in that** the position (3) of a material-removing effector geometry (2) is detected quasi-continuously and stored together with the time of measurement, said material-removing effector geometry being known and/or measured, acquired and stored together with the time of measurement and/or the position of the effector volume either on demand or quasi-continuously.

7. The method according to any of claims 1 to 6, **characterized in that** an effective volume is calculated, either on demand or quasi-continuously, from the spatial overlap of the effector volume in the detected positions and, preferably on demand or quasi-continuously, an intersection volume is formed from the intersection of the effective volume and an object volume, or the geometrical description thereof is calculated, and wherein the position of the object volume relative to the effective volume is known or measured and, preferably on demand or quasi-continuously, the intersection volume description is used to calculate the geometry of a fitting body, and the geometry of the fitting body can be suitably changed so as to meet additional medical criteria, or criteria for the production or integration of the fitting body, the intersection volume description in the simplest case corresponding to the shape of the fitting body.

8. The method according to claim 1, **characterized in that** at least one table including at least one geometrical description of a fitting body as standard body is used and/or that a standard body meeting an optimization criterion is selected from a standard body table, the standard body in the simplest case corresponding to a single entry of a fitting body in the table, and a suitable store with prefabricated standard bodies corresponding to the table of standard bodies being available, which do not require further processing or only to a minor extent.

9. The method according to any of the preceding claims, **characterized in that** the difference volume between the selected or actually calculated fitting body and the actual intersection volume is calculated and/or that the effector energy is switched off if the effector is outside the geometry of the fitting body and/or that the effector energy is switched on if the effector is within the geometry of the fitting body.

10. The method according to any of the preceding claims, **characterized in that** a soft object is stabilized in a largely dimensionally stable manner by a template which offers at least one possible way of conducting or passing material-removing energy or a material-removing effector therethrough in a defined manner, and that the difference volume geometry is used to arrange and guide the effector and/or that removed particles, vapors and odors formed during removal are sucked off.

11. The method according to claim 1, **characterized in that** the effector is used not only for removal of material, but also in 3D surface geometry measurement.

12. The method according to claim 1, **characterized in that** the effector is a laser and the tissue-removing effector geometry is achieved by means of a replaceable light-transmitting positive effector geometry body and/or a light-impermeable negative effector geometry body which allows the preparation of, for example, undercut cavities.

13. The method according to claim 1, **characterized in that** the object geometry is detected directly by means of a volume imaging method (X-ray, MRT, CT, ultrasound, etc.) or a surface imaging method (3D surface scanner, manual scanner, tactile sensor) or via an impression and calculated and/or that initial removal of material or tissue is performed on a model and the detected position and shape of the removal of material or tissue is used for the removal on the same model, other models as well as in patients or on objects.

14. The method according to claim 1, **characterized in that** the removal of material or tissue detected via the effective, intersection or difference geometries or by means of a 3D surface scanner is stored as geometrical model and used in a further material or tissue removal operation on the same or other objects or models, it also being possible to mirror positive geometries into negative geometries so as to suitably cut out for cavities, transplants.

15. The method according to any of the preceding claims, **characterized in that** the position and geometry of the material or tissue (13) yet to remove is calculated on the basis of the position and geometry of material or tissue (10) already removed and of a defined position and defined geometry of an object shape (12) to be achieved, displayed graphically on a screen, and the information relating to the position (3) of the effector (2) relative to the boundary surface between the object fitting shape to be achieved and material or tissue (13) yet to remove is similarly displayed graphically and/or acoustically and used for manual and/or kinematic arrangement and guidance of the effector, the processed information being outputable via an interface to address a kinematic system of arrangement and guidance for the effector.

16. The method according to claim 1, **characterized in that** the working volume is defined such that it need not be abandoned during normal use of the effector (2), but typically has to be abandoned for tool replacement.

17. A device system for removing material or tissue or for working material or tissue by means of at least one manually guided effector, using a navigation system and utilizing computer technology, wherein a first marking support (6) with markings (7) is arranged on a handpiece (1) with effector (2), and the handpiece (1) is connected to a control unit (22), and a second marking support (6) with markings (7) is attached to the material object or tissue object, and the control unit (22) is connected to a position measuring system (16), **characterized in that** the control unit is configured to control or regulate the power of the material-removing effector (2) via the control unit (22) in accordance with the object shape to be achieved and on the basis of the distance from the effector position (3) and/or effector geometry to the boundary surface between the material (13) to be removed and object shape to be achieved and switch off at the latest when there is overlapping between removed material (10) and object shape (12) to be achieved.

18. The device system according to claim 17, **characterized in that** the control unit (22) is connected to a screen, and a power control device (18), a power converter (19) or a throttle device (20) with throttle interface (21) is arranged between control unit (22) and hand piece (1), and that the second marking support (6) has a calibration device (24).

19. Use of the device system according to claim 17 for the removal of tissue in medicine and dentistry.

20. Use of the device system according to claim 17 for the removal of material from metal, glass, ceramics, wood, plastics in various fields of use and for model works.

## Revendications

1. Procédé d'enlèvement de matière ou de travail de matière au moyen d'au moins un effecteur guidé manuellement en faisant appel à un système de navigation et en utilisant une technique informatique,
dans lequel des données se rapportant au positionnement et à l'orientation de l'effecteur et leurs modifications par rapport à la position d'au moins un corps de référence sont recueillies, enregistrées et traitées par informatique et déclenchent des instructions de commande et/ou de régulation,
**caractérisé en ce que** l'effecteur est commuté en fonctionnement en service ou hors service en fonction d'un volume de travail et/ou d'un volume d'enlèvement de matière et/ou d'un volume de résidu de matière prescrit et il est commandé et/ou réglé en service dans sa puissance et/ou sa paramétrisation et **en ce que** la puissance de l'effecteur (2) enlevant de la matière est mise hors circuit au plus tard lorsqu'il y a un chevauchement entre la matière enlevée (10) et la forme d'objet (12) à atteindre.

2. Procédé selon la revendication 1, **caractérisé en ce que** la position et la géométrie de la surface d'objet obtenue sont détectées et enregistrées pour d'autres opérations de travail sur le même objet ou d'autres objets.

3. Procédé selon la revendication 1, **caractérisé en ce que** la position (3) d'une géométrie d'effecteur (2) enlevant de la matière par rapport à la position (4) d'un objet (5) est détectée de manière quasi-continue durant une période de temps définie et la géométrie (10) de la matière enlevée est calculée à partir de là, la géométrie (10) de la matière enlevée étant calculée en intégrant des données d'image de surface d'un scanner 3D.

4. Procédé selon la revendication 1, **caractérisé en ce que** la position et la géométrie d'une matière encore à enlever (13) sont calculées sur la base d'une position définie et d'une géométrie définie d'une forme d'objet (12) à obtenir et/ou sur la base de la position et de la géométrie du tissu (10) déjà enlevé et utilisées pour agencer et guider l'effecteur et **en ce que** la puissance de l'effecteur enlevant de la matière est commandée et/ou régulée au moins dans la géométrie de la matière encore à enlever (12) en se basant sur la distance de la position d'effecteur (3) et/ou de la géométrie d'effecteur (2) à la surface limite entre la matière à enlever (13) et la forme d'objet à atteindre (12).

5. Procédé selon l'une quelconque des revendications 1 à 4, **caractérisé en ce que** la puissance d'enlèvement de matière est délivrée par l'intermédiaire d'une matrice de façonnage transportant de l'énergie sans modifier la position de l'effecteur (2) directement pour obtenir des parties de la forme ajustée de l'objet et/ou **en ce que** la forme d'objet (12) à atteindre est calculée sur la base de critères médicaux et/ou de matière déjà enlevée et/ou de critères de fabrication de forme d'objet et de corps ajusté et/ou de critères d'intégration de forme d'objet et de corps ajusté et **en ce que** la géométrie du corps ajusté est utilisée pour mesurer une quantité de matière et/ou pour fabriquer un corps ajusté.

6. Procédé selon la revendication 5, **caractérisé en ce que** la position (3) d'une géométrie d'effecteur (2) enlevant de la matière est détectée de manière quasi-continue et est enregistrée avec l'instant de mesure, la géométrie d'effecteur enlevant de la matière étant connue et/ou mesurée, acquise et enregistrée avec l'instant de mesure et/ou la position du volume d'effecteur, sur demande ou de manière quasi-continue.

7. Procédé selon l'une quelconque des revendications 1 à 6, **caractérisé en ce qu'**un volume effectif est calculé sur demande ou de manière quasi-continue à partir du recouvrement spatial du volume d'effecteur dans les positions détectées et de préférence sur demande ou de manière quasi-continue, un volume d'intersection est constitué à partir de l'intersection du volume effectif et d'un volume d'objet, ou sa description géométrique est calculée, et dans lequel la position du volume d'objet par rapport au volume effectif est connue ou est mesurée et, de préférence sur demande ou de manière quasi-continue, la description du volume d'intersection est utilisée pour calculer la géométrie d'un corps ajusté, et la géométrie du corps ajusté peut être modifiée de façon appropriée pour satisfaire à des critères médicaux additionnels ou des critères de fabrication ou d'intégration du corps ajusté, la description du volume d'intersection dans le cas le plus simple correspondant à la forme du corps ajusté.

8. Procédé selon la revendication 1, **caractérisé en ce qu'**au moins une table comportant au moins une description géométrique d'un corps ajusté à titre de corps standard est utilisée et/ou **en ce qu'**un corps standard répondant à un critère d'optimisation est sélectionné à partir d'une table de corps standards, le corps standard correspondant dans le cas le plus simple à un unique enregistrement d'un corps ajusté dans la table et il existe un stock de corps standards déjà préfabriqués correspondant à la table des corps ajustés, qui ne doivent pas être retravaillés ou doivent l'être seulement dans une faible mesure.

9. Procédé selon l'une quelconque des revendications précédentes, **caractérisé en ce que** le volume différentiel entre le corps ajusté sélectionné ou calculé à ce moment et le volume d'intersection en cours est calculé et/ou l'énergie de l'effecteur est mise hors circuit lorsque l'effecteur se trouve à l'extérieur de la géométrie du corps ajusté et/ou l'énergie de l'effecteur est mise en circuit lorsque l'effecteur se trouve à l'intérieur de la géométrie du corps ajusté.

10. Procédé selon l'une quelconque des revendications précédentes, **caractérisé en ce qu'**un objet tendre, essentiellement stable dans ses dimensions, est stabilisé par une matrice, qui offre au moins une possibilité de transmission ou de passage défini d'énergie enlevant de la matière ou d'un effecteur enlevant de la matière et **en ce que** la géométrie de volume différentiel est utilisée pour agencer et guider l'effecteur et/ou **en ce que** les particules produites par l'enlèvement, les vapeurs et les odeurs sont aspirées au cours de l'enlèvement.

11. Procédé selon la revendication 1, **caractérisé en ce que** l'effecteur est utilisé non seulement pour enlever de la matière, mais aussi pour mesurer la géométrie de surface en 3D.

12. Procédé selon la revendication 1, **caractérisé en ce que** l'effecteur est un laser et la géométrie d'effecteur enlevant du tissu est obtenue par un corps de géométrie d'effecteur positive guidant la lumière, interchangeable, et/ou un corps de géométrie d'effecteur négative, opaque, avec lequel des cavités de contre-dépouille par exemple peuvent être fabriquées.

13. Procédé selon la revendication 1, **caractérisé en ce que** la géométrie de l'objet est détectée et calculée directement par un procédé fournissant une image de volume (rayons X, MRT, CT, ultrasons, etc.) ou par un procédé d'imagerie de surface (scanner de surface en 3D, scanner manuel, capteur tactile) ou par l'intermédiaire d'une empreinte et/ou **en ce qu'**un premier enlèvement de matière ou de tissu est effectué sur un modèle et la position et la forme détectées d'enlèvement de matière ou de tissu sont utilisées pour l'enlèvement sur le même modèle, d'autres modèles ainsi que sur des patients ou des objets.

14. Procédé selon la revendication 1, **caractérisé en ce que** l'enlèvement de matière ou de tissu, détectée par l'intermédiaire de la géométrie effective, d'intersection ou différentielle ou par un scanner de surface en 3D, est enregistré sous forme de modèle géométrique, et est utilisé pour une autre opération d'enlèvement de matière ou de tissu sur le même ou d'autres objets ou modèles, moyennant quoi des géométries positives peuvent également être reflétées en géométries négatives, pour effectuer un évidement par exemple de manière appropriée pour des cavités, des greffons.

15. Procédé selon l'une quelconque des revendications précédentes, **caractérisé en ce que** la position et la géométrie de la matière ou du tissu (13) encore à enlever sont calculées sur la base de la position et de la géométrie de la matière ou du tissu (10) déjà enlevé et d'une position définie et d'une géométrie définie d'une forme d'objet (12) à atteindre, représentées graphiquement sur un écran, et les informations sur la position (3) de l'effecteur (2) à la surface limite entre la forme d'objet ajusté à atteindre et la matière ou le tissu (13) encore à enlever sont également représentées graphiquement et/ou acoustiquement et sont utilisées pour l'agencement et la conduite manuelle et/ou cinématique de l'effecteur, les informations traitées pouvant être débitées par l'intermédiaire d'une interface pour exciter un dispositif cinématique d'agencement et de pilotage d'effecteur.

16. Procédé selon la revendication 1, **caractérisé en ce que** le volume de travail est défini de telle sorte qu'il ne doit pas être abandonné en cas d'utilisation normale de l'effecteur (2), mais qu'il est généralement abandonné en cas de changement d'outil.

17. Système d'appareils d'enlèvement de matière ou de tissu ou de travail de matière ou de tissu au moyen d'au moins un effecteur piloté manuellement en faisant appel à un système de navigation et en utilisant une technique informatique,
dans lequel un premier support de repérage (6) comportant des repérages (7) est disposé sur un porte-outil (1) comportant un effecteur (2) et le porte-outil (1) est relié à une unité de commande (22), et un second support de repérage (6) comportant des repérages (7) est fixé à l'objet de matière ou de tissu, l'unité de commande (22) étant reliée à un système de mesure de position (16),
**caractérisé en ce que** l'unité de commande est configurée de sorte à commander et réguler la puissance de l'effecteur (2) enlevant de la matière, par l'intermédiaire de l'unité de commande (22), en fonction de la forme d'objet à obtenir et sur la base de la distance de la position de l'effecteur (3) et/ou de la géométrie d'effecteur à la surface limite entre la matière à enlever (13) et à la forme d'objet à atteindre et à mettre hors service la puissance au plus tard lorsqu'il se produit un chevauchement entre la matière enlevée (10) et à la forme d'objet (12) à atteindre.

18. Système d'appareils selon la revendication 17, **caractérisé en ce que** l'unité de commande (22) est connectée à un écran, et un organe de commande de puissance (18), un convertisseur de puissance (19) ou un dispositif d'étranglement (20) comportant une interface d'étranglement (21) est disposé entre l'unité de commande (22) et le porte-outil (1) et **en ce que** le second support de repérage (6) comprend un dispositif d'étalonnage (24).

19. Utilisation du système d'appareils selon la revendication 17 pour l'enlèvement de tissu en médecine ou en odontologie.

20. Utilisation du système d'appareils selon la revendication 17 pour l'enlèvement de matière à partir de métal, de verre, de céramique, de bois, de matière plastique dans différents domaines d'application et pour le travail de modèles.
